# EUROPEAN PATENT SPECIFICATION

(11) **EP 2 009 099 B1**
(45) Date of publication and mention of the grant of the patent: **06.04.2011**
(21) Application number: 07012583.6
(22) Date of filing: 27.06.2007
(51) Int. Cl.: C12N 9/78

(54) **Modulation of production of retroviruses by APOBEC4**
Modulation der Erzeugung von Retroviren durch APOBEC4
Modulation de la production de rétrovirus par APOBEC4

(43) Date of publication of application: 31.12.2008
(73) Proprietor: Bundesrepublik Deutschland, letztvertreten durch den Präsidenten des Paul-Ehrlich-Instituts Prof. Dr. Johannes Löwer, 63225 Langen (DE)
(72) Inventor: Perkovic, Mario, 40591 Düsseldorf (DE); Münk, Carsten, 60323 Frankfurt (DE); Cichutek, Klaus, 63225 Langen (DE); Marino, Daniela, 40591 Düsseldorf (DE)
(74) Representative: Grund, Martin

(56) References cited:
- WO-A-2005/117947
- WO-A-2006/065377
- ROGOZIN IGOR B ET AL: "APOBEC4, a new member of the AID/APOBEC family of polynucleotide (deoxy)cytidine deaminases predicted by computational analysis." CELL CYCLE (GEORGETOWN, TEX.) SEP 2005, vol. 4, no. 9, September 2005 (2005-09), pages 1281-1285, XP002457059 ISSN: 1551-4005
- HOLMES ET AL: "APOBEC-mediated viral restriction: not simply editing?" TRENDS IN BIOCHEMICAL SCIENCES, ELSEVIER, HAYWARDS, GB, vol. 32, no. 3, 12 March 2007 (2007-03-12), pages 118-128, XP005922435 ISSN: 0968-0004

### SEQUENCE LISTING

<110> Bundesrepublik Deutschland, letztvertreten durch den Präsidenten des Paul-Ehrlich-Instituts Prof. Dr. Johannes Löwer
<120> Modulation of Production of Retroviruses by APOBEC4
<130> 141-003
<160> 12
<170> PatentIn version 3.4
<210> 1
   <211> 5255
   <212> DNA
   <213> Plasmid phA-3xHA
<220>
   <221> promoter
   <222> (3)..(591)
   <223> CMV
<220>
   <221> CDS
   <222> (702)..(1919)
   <223> hAPOBEC4-3xHA
<220>
   <221> misc_feature
   <222> (1827)..(1853)
   <223> HA1
<220>
   <221> misc_feature
   <222> (1857)..(1883)
   <223> HA2
<220>
   <221> misc_feature
   <222> (1887)..(1913)
   <223> HA3
<220>
   <221> misc_feature
   <222> (1917)..(1919)
   <223> STOP
<220>
   <221> polyA_signal
   <222> (2076)..(2126)
   <223> SV40 poly A
<220>
   <221> misc_feature
   <222> (3153)..(3947)
   <223> Kan/Neo r
<400> 1
   attagttatt aatagtaatc aattacgggg tcattagttc atagcccata tatggagttc     60
   cgcgttacat aacttacggt aaatggcccg cctggctgac cgcccaacga cccccgccca    120
   ttgacgtcaa taatgacgta tgttcccata gtaacgccaa tagggacttt ccattgacgt    180
   caatgggtgg agtatttacg gtaaactgcc cacttggcag tacatcaagt gtatcatatg    240
   ccaagtacgc cccctattga cgtcaatgac ggtaaatggc ccgcctggca ttatgcccag    300
   tacatgacct tatgggactt tcctacttgg cagtacatct acgtattagt catcgctatt    360
   accatggtga tgcggttttg gcagtacatc aatgggcgtg gatagcggtt tgactcacgg    420
   ggatttccaa gtctccaccc cattgacgtc aatgggagtt tgttttggca ccaaaatcaa    480
   cgggactttc caaaatgtcg taacaactcc gccccattga cgcaaatggg cggtaggcgt    540
   gtacggtggg aggtctatat aagcagagct ggtttagtga accgtcagat ccgctagcgc    600
   taccggactc agatctcgag ctcaagcttc gaattcgccc ttcaggcggt acagcctgga    660
   gacaattgat ggtttgcagc tagaagacag taaatctagc a atg gag ccc ata tat    716
   gag gag tac cta gca aat cat gga aca ata gta aaa cca tat tac tgg      764
   cta agc ttc tct ctc gat tgc tct aat tgt cct tac cat att cga aca      812
   ggt gaa gaa gca aga gtt tcc ctc aca gaa ttt tgt cag att ttt gga      860
   ttc cct tat ggg aca aca ttt cct caa aca aaa cac ctc aca ttt tat      908
   gaa cta aaa act tct tct ggt agc ctg gtg caa aag ggc cat gct agc      956
   agt tgc act ggg aat tat atc cat cca gaa tca atg ctc ttt gaa atg     1004
   aat ggt tat ctt gac tca gcc ata tac aat aat gac agc atc agg cat     1052
   atc att ctg tat tcc aac aac tcc cct tgt aat gaa gct aac cac tgc     1100
   tgc atc agc aaa atg tat aat ttc ctg att acg tat cca ggc atc act      1148
   ctt agt att tat ttt tct cag ctc tat cat act gag atg gac ttt cct      1196
   gcc tca gca tgg aac cgc gaa gct ctc cgg agc ctg gcc agc tta tgg      1244
   ccg cgg gtt gtt ttg agt cca ata agt ggt ggg atc tgg cat tct gtt      1292
   ctc cac agc ttt ata agt ggt gtc tca gga tca cat gtt ttt cag ccc      1340
   att tta act ggg aga gca ctg gct gac agg cac aac gca tat gaa atc      1388
   aat gcc ata aca ggc gta aaa cct tac ttc act gat gtt ctt ctc cag      1436
   aca aaa agg aat cca aac aca aaa gct cag gag gct tta gag agc tac      1484
   ccc tta aac aat gcc ttt cct gga cag ttt ttt caa atg ccg agt gga      1532
   caa ctc caa ccc aac cta cct cca gac ctc agg gct cct gtt gtt ttt      1580
   gtg cta gtg cct ctc agg gac tta cca cca atg cat atg ggc caa aac      1628
   cca aat aaa ccc agg aat atc gta agg cac tta aat atg cct caa atg      1676
   tca ttc cag gaa acc aag gac ctt gga agg ctt ccc act gga agg tca      1724
   gtg gag ata gtg gaa atc aca gaa cag ttt gca agt agc aag gag gca      1772
   gat gaa aag aag aag aag aaa ggg aag aaa ggg gat cca ccg gtc gcc     1820
   acc atg tac ccc tac gac gtg ccc gac tac gcc ggc tac ccc tac gac     1868
   gtg ccc gac tac gcc ggc tac ccc tac gac gtg ccc gac tac gcc ggc     1916
   taa ctgagcggcc gcgactctag atcataatca gccataccac atttgtagag          1969
   gttttacttg ctttaaaaaa cctcccacac ctccccctga acctgaaaca taaaatgaat   2029
   gcaattgttg ttgttaactt gtttattgca gcttataatg gttacaaata aagcaatagc   2089
   atcacaaatt tcacaaataa agcatttttt tcactgcatt ctagttgtgg tttgtccaaa   2149
   ctcatcaatg tatcttaagg cgtaaattgt aagcgttaat attttgttaa aattcgcgtt   2209
   aaatttttgt taaatcagct cattttttaa ccaataggcc gaaatcggca aaatccctta   2269
   taaatcaaaa gaatagaccg agatagggtt gagtgttgtt ccagtttgga acaagagtcc   2329
   actattaaag aacgtggact ccaacgtcaa agggcgaaaa accgtctatc agggcgatgg   2389
   cccactacgt gaaccatcac cctaatcaag ttttttgggg tcgaggtgcc gtaaagcact   2449
   aaatcggaac cctaaaggga gcccccgatt tagagcttga cggggaaagc cggcgaacgt   2509
   ggcgagaaag gaagggaaga aagcgaaagg agcgggcgct agggcgctgg caagtgtagc   2569
   ggtcacgctg cgcgtaacca ccacacccgc cgcgcttaat gcgccgctac agggcgcgtc   2629
   aggtggcact tttcggggaa atgtgcgcgg aacccctatt tgtttatttt tctaaataca   2689
   ttcaaatatg tatccgctca tgagacaata accctgataa atgcttcaat aatattgaaa   2749
   aaggaagagt cctgaggcgg aaagaaccag ctgtggaatg tgtgtcagtt agggtgtgga   2809
   aagtccccag gctccccagc aggcagaagt atgcaaagca tgcatctcaa ttagtcagca   2869
   accaggtgtg gaaagtcccc aggctcccca gcaggcagaa gtatgcaaag catgcatctc   2929
   aattagtcag caaccatagt cccgccccta actccgccca tcccgcccct aactccgccc   2989
   agttccgccc attctccgcc ccatggctga ctaatttttt ttatttatgc agaggccgag   3049
   gccgcctcgg cctctgagct attccagaag tagtgaggag gcttttttgg aggcctaggc   3109
   ttttgcaaag atcgatcaag agacaggatg aggatcgttt cgcatgattg aacaagatgg   3169
   attgcacgca ggttctccgg ccgcttgggt ggagaggcta ttcggctatg actgggcaca   3229
   acagacaatc ggctgctctg atgccgccgt gttccggctg tcagcgcagg ggcgcccggt   3289
   tctttttgtc aagaccgacc tgtccggtgc cctgaatgaa ctgcaagacg aggcagcgcg 3349
   gctatcgtgg ctggccacga cgggcgttcc ttgcgcagct gtgctcgacg ttgtcactga 3409
   agcgggaagg gactggctgc tattgggcga agtgccgggg caggatctcc tgtcatctca 3469
   ccttgctcct gccgagaaag tatccatcat ggctgatgca atgcggcggc tgcatacgct 3529
   tgatccggct acctgcccat tcgaccacca agcgaaacat cgcatcgagc gagcacgtac 3589
   tcggatggaa gccggtcttg tcgatcagga tgatctggac gaagagcatc aggggctcgc 3649
   gccagccgaa ctgttcgcca ggctcaaggc gagcatgccc gacggcgagg atctcgtcgt 3709
   gacccatggc gatgcctgct tgccgaatat catggtggaa aatggccgct tttctggatt 3769
   catcgactgt ggccggctgg gtgtggcgga ccgctatcag gacatagcgt tggctacccg 3829
   tgatattgct gaagagcttg gcggcgaatg ggctgaccgc ttcctcgtgc tttacggtat 3889
   cgccgctccc gattcgcagc gcatcgcctt ctatcgcctt cttgacgagt tcttctgagc 3949
   gggactctgg ggttcgaaat gaccgaccaa gcgacgccca acctgccatc acgagatttc 4009
   gattccaccg ccgccttcta tgaaaggttg ggcttcggaa tcgttttccg ggacgccggc 4069
   tggatgatcc tccagcgcgg ggatctcatg ctggagttct tcgcccaccc tagggggagg 4129
   ctaactgaaa cacggaagga gacaataccg gaaggaaccc gcgctatgac ggcaataaaa 4189
   agacagaata aaacgcacgg tgttgggtcg tttgttcata aacgcggggt tcggtcccag 4249
   ggctggcact ctgtcgatac cccaccgaga ccccattggg gccaatacgc ccgcgtttct 4309
   tccttttccc caccccaccc cccaagttcg ggtgaaggcc cagggctcgc agccaacgtc 4369
   ggggcggcag gccctgccat agcctcaggt tactcatata tactttagat tgatttaaaa 4429
   cttcattttt aatttaaaag gatctaggtg aagatccttt ttgataatct catgaccaaa 4489
   atcccttaac gtgagttttc gttccactga gcgtcagacc ccgtagaaaa gatcaaagga 4549
   tcttcttgag atcctttttt tctgcgcgta atctgctgct tgcaaacaaa aaaaccaccg 4609
   ctaccagcgg tggtttgttt gccggatcaa gagctaccaa ctctttttcc gaaggtaact 4669
   ggcttcagca gagcgcagat accaaatact gtccttctag tgtagccgta gttaggccac 4729
   cacttcaaga actctgtagc accgcctaca tacctcgctc tgctaatcct gttaccagtg 4789
   gctgctgcca gtggcgataa gtcgtgtctt accgggttgg actcaagacg atagttaccg 4849
   gataaggcgc agcggtcggg ctgaacgggg ggttcgtgca cacagcccag cttggagcga 4909
   acgacctaca ccgaactgag atacctacag cgtgagctat gagaaagcgc cacgcttccc 4969
   gaagggagaa aggcggacag gtatccggta agcggcaggg tcggaacagg agagcgcacg 5029
   agggagcttc cagggggaaa cgcctggtat ctttatagtc ctgtcgggtt tcgccacctc 5089
   tgacttgagc gtcgattttt gtgatgctcg tcaggggggc ggagcctatg gaaaaacgcc 5149
   agcaacgcgg cctttttacg gttcctggcc ttttgctggc cttttgctca catgttcttt 5209
   cctgcgttat cccctgattc tgtggataac cgtattaccg ccatgc 5255
<210> 2
   <211> 405
   <212> PRT
   <213> Plasmid phA-3xHA
<400> 2
<210> 3
   <211> 6138
   <212> DNA
   <213> Plasmid pHA-hA4-cDNA3.1Zeo(+)
<220>
   <221> promoter
   <222> (232)..(819)
   <223> CMV promoter
<220>
   <221> primer_bind
   <222> (769)..(789)
   <223> CMV forward primer
<220>
   <221> primer_bind
   <222> (863)..(882)
   <223> T7 primer
<220>
   <221> promoter
   <222> (863)..(879)
   <223> T7 promoter
<220>
   <221> CDS
   <222> (941)..(2074)
   <223> HA-hA4
<220>
   <221> misc_feature
   <222> (947)..(973)
   <223> HA-Tag
<220>
   <221> primer_bind
   <222> (2145)..(2162)
   <223> BGH reverse primer
<220>
   <221> polyA_signal
   <222> (2151)..(2375)
   <223> BGH pA
<220>
   <221> rep_origin
   <222> (2421)..(2849)
   <223> f1 origin
<220>
   <221> promoter
   <222> (2854)..(3224)
   <223> SV40 early promoter
<220>
   <221> promoter
   <222> (3239)..(3306)
   <223> EM7 promoter
<220>
   <221> misc_feature
   <222> (3307)..(3681)
   <223> Zeo(R)
<220>
   <221> rep_origin
   <222> (4324)..(4997)
   <223> pUC origin
<220>
   <221> misc_feature
   <222> (5142)..(6002)
   <223> Amp(R)
<220>
   <221> promoter
   <222> (6003)..(6101)
   <223> bla promoter
<400> 3
   gacggatcgg gagatctccc gatcccctat ggtcgactct cagtacaatc tgctctgatg     60
   ccgcatagtt aagccagtat ctgctccctg cttgtgtgtt ggaggtcgct gagtagtgcg    120
   cgagcaaaat ttaagctaca acaaggcaag gcttgaccga caattgcatg aagaatctgc    180
   ttagggttag gcgttttgcg ctgcttcgcg atgtacgggc cagatatacg cgttgacatt    240
   gattattgac tagttattaa tagtaatcaa ttacggggtc attagttcat agcccatata    300
   tggagttccg cgttacataa cttacggtaa atggcccgcc tggctgaccg cccaacgacc    360
   cccgcccatt gacgtcaata atgacgtatg ttcccatagt aacgccaata gggactttcc    420
   attgacgtca atgggtggac tatttacggt aaactgccca cttggcagta catcaagtgt    480
   atcatatgcc aagtacgccc cctattgacg tcaatgacgg taaatggccc gcctggcatt    540
   atgcccagta catgacctta tgggactttc ctacttggca gtacatctac gtattagtca    600
   tcgctattac catggtgatg cggttttggc agtacatcaa tgggcgtgga tagcggtttg    660
   actcacgggg atttccaagt ctccacccca ttgacgtcaa tgggagtttg ttttggcacc    720
   aaaatcaacg ggactttcca aaatgtcgta acaactccgc cccattgacg caaatgggcg    780
   gtaggcgtgt acggtgggag gtctatataa gcagagctct ctggctaact agagaaccca    840
   ctgcttactg gcttatcgaa attaatacga ctcactatag ggagacccaa gctggctagc    900
   gtttaaactt aagcttggta ccgagctcgg atccctagca atg gga tat cca tac      955
                                               Met Gly Tyr Pro Tyr
                                               1               5
   gat gtt cca gat tac gct gag ccc ata tat gag gag tac cta gca aat     1003
   Asp Val Pro Asp Tyr Ala Glu Pro Ile Tyr Glu Glu Tyr Leu Ala Asn
                   10                  15                  20
   cat gga aca ata gta aaa cca tat tac tgg cta agc ttc tct ctc gat     1051
   His Gly Thr Ile Val Lys Pro Tyr Tyr Trp Leu Ser Phe Ser Leu Asp
               25                  30                  35
   tgc tct aat tgt cct tac cat att cga aca ggt gaa gaa gca aga gtt     1099
   Cys Ser Asn Cys Pro Tyr His Ile Arg Thr Gly Glu Glu Ala Arg Val
           40                  45                  50
   tcc ctc aca gaa ttt tgt cag att ttt gga ttc cct tat ggg aca aca     1147
   Ser Leu Thr Glu Phe Cys Gln Ile Phe Gly Phe Pro Tyr Gly Thr Thr
       55                  60                  65
   ttt cct caa aca aaa cac ctc aca ttt tat gaa cta aaa act tct tct     1195
   Phe Pro Gln Thr Lys His Leu Thr Phe Tyr Glu Leu Lys Thr Ser Ser
   70                  75                  80                  85
   ggt agc ctg gtg caa aag ggc cat gct agc agt tgc act ggg aat tat     1243
   Gly Ser Leu Val Gln Lys Gly His Ala Ser Ser Cys Thr Gly Asn Tyr
                   90                  95                  100
   atc cat cca gaa tca atg ctc ttt gaa atg aat ggt tat ctt gac tca     1291
   Ile His Pro Glu Ser Met Leu Phe Glu Met Asn Gly Tyr Leu Asp Ser
               105                 110                 115
   gcc ata tac aat aat gac agc atc agg cat atc att ctg tat tcc aac     1339
   Ala Ile Tyr Asn Asn Asp Ser Ile Arg His Ile Ile Leu Tyr Ser Asn
           120                 125                 130
   aac tcc cct tgt aat gaa gct aac cac tgc tgc atc agc aaa atg tat     1387
   Asn Ser Pro Cys Asn Glu Ala Asn His Cys Cys Ile Ser Lys Met Tyr
       135                 140                 145
   aat ttc ctg att acg tat cca ggc atc act ctt agt att tat ttt tct     1435
   Asn Phe Leu Ile Thr Tyr Pro Gly Ile Thr Leu Ser Ile Tyr Phe Ser
   150                 155                 160                 165
   cag ctc tat cat act gag atg gac ttt cct gcc tca gca tgg aac cgc     1483
   Gln Leu Tyr His Thr Glu Met Asp Phe Pro Ala Ser Ala Trp Asn Arg
                   170                 175                 180
   gaa gct ctc cgg agc ctg gcc agc tta tgg ccg cgg gtt gtt ttg agt     1531
   Glu Ala Leu Arg Ser Leu Ala Ser Leu Trp Pro Arg Val Val Leu Ser
               185                 190                 195
   cca ata agt ggt ggg atc tgg cat tct gtt ctc cac agc ttt ata agt     1579
   Pro Ile Ser Gly Gly Ile Trp His Ser Val Leu His Ser Phe Ile Ser
           200                 205                 210
   ggt gtc tca gga tca cat gtt ttt cag ccc att tta act ggg aga gca     1627
   Gly Val Ser Gly Ser His Val Phe Gln Pro Ile Leu Thr Gly Arg Ala
       215                 220                 225
   ctg gct gac agg cac aac gca tat gaa atc aat gcc ata aca ggc gta      1675
   Leu Ala Asp Arg His Asn Ala Tyr Glu Ile Asn Ala Ile Thr Gly Val
   230                 235                 240                 245
   aaa cct tac ttc act gat gtt ctt ctc cag aca aaa agg aat cca aac      1723
   Lys Pro Tyr Phe Thr Asp Val Leu Leu Gln Thr Lys Arg Asn Pro Asn
                   250                 255                 260
   aca aaa gct cag gag gct tta gag agc tac ccc tta aac aat gcc ttt      1771
   Thr Lys Ala Gln Glu Ala Leu Glu Ser Tyr Pro Leu Asn Asn Ala Phe
               265                 270                 275
   cct gga cag ttt ttt caa atg ccg agt gga caa ctc caa ccc aac cta      1819
   Pro Gly Gln Phe Phe Gln Met Pro Ser Gly Gln Leu Gln Pro Asn Leu
           280                 285                 290
   cct cca gac ctc agg gct cct gtt gtt ttt gtg cta gtg cct ctc agg      1867
   Pro Pro Asp Leu Arg Ala Pro Val Val Phe Val Leu Val Pro Leu Arg
       295                 300                 305
   gac tta cca cca atg cat atg ggc caa aac cca aat aaa ccc agg aat      1915
   Asp Leu Pro Pro Met His Met Gly Gln Asn Pro Asn Lys Pro Arg Asn
   310                 315                 320                 325
   atc gta agg cac tta aat atg cct caa atg tca ttc cag gaa acc aag      1963
   Ile Val Arg His Leu Asn Met Pro Gln Met Ser Phe Gln Glu Thr Lys
                   330                 335                 340
   gac ctt gga agg ctt ccc act gga agg tca gtg gag ata gtg gaa atc      2011
   Asp Leu Gly Arg Leu Pro Thr Gly Arg Ser Val Glu Ile Val Glu Ile
               345                 350                 355
   aca gaa cag ttt gca agt agc aag gag gca gat gaa aag aag aag aag      2059
   Thr Glu Gln Phe Ala Ser Ser Lys Glu Ala Asp Glu Lys Lys Lys Lys
           360                 365                 370
   aaa ggg aag aaa taa gaattctgca gatatccagc acagtggcgg ccgctcgagt      2114
   Lys Gly Lys Lys
       375
   ctagagggcc cgtttaaacc cgctgatcag cctcgactgt gccttctagt tgccagccat    2174
   ctgttgtttg cccctccccc gtgccttcct tgaccctgga aggtgccact cccactgtcc    2234
   tttcctaata aaatgaggaa attgcatcgc attgtctgag taggtgtcat tctattctgg    2294
   ggggtggggt ggggcaggac agcaaggggg aggattggga agacaatagc aggcatgctg    2354
   gggatgcggt gggctctatg gcttctgagg cggaaagaac cagctggggc tctagggggt    2414
   atccccacgc gccctgtagc ggcgcattaa gcgcggcggg tgtggtggtt acgcgcagcg    2474
   tgaccgctac acttgccagc gccctagcgc ccgctccttt cgctttcttc ccttcctttc    2534
   tcgccacgtt cgccggcttt ccccgtcaag ctctaaatcg gggcatccct ttagggttcc     2594
   gatttagtgc tttacggcac ctcgacccca aaaaacttga ttagggtgat ggttcacgta   2654
   gtgggccatc gccctgatag acggtttttc gccctttgac gttggagtcc acgttcttta   2714
   atagtggact cttgttccaa actggaacaa cactcaaccc tatctcggtc tattcttttg   2774
   atttataagg gattttgggg atttcggcct attggttaaa aaatgagctg atttaacaaa   2834
   aatttaacgc gaattaattc tgtggaatgt gtgtcagtta gggtgtggaa agtccccagg   2894
   ctccccaggc aggcagaagt atgcaaagca tgcatctcaa ttagtcagca accaggtgtg   2954
   gaaagtcccc aggctcccca gcaggcagaa gtatgcaaag catgcatctc aattagtcag   3014
   caaccatagt cccgccccta actccgccca tcccgcccct aactccgccc agttccgccc   3074
   attctccgcc ccatggctga ctaatttttt ttatttatgc agaggccgag gccgcctctg   3134
   cctctgagct attccagaag tagtgaggag gcttttttgg aggcctaggc ttttgcaaaa   3194
   agctcccggg agcttgtata tccattttcg gatctgatca gcacgtgttg acaattaatc   3254
   atcggcatag tatatcggca tagtataata cgacaaggtg aggaactaaa ccatggccaa   3314
   gttgaccagt gccgttccgg tgctcaccgc gcgcgacgtc gccggagcgg tcgagttctg   3374
   gaccgaccgg ctcgggttct cccgggactt cgtggaggac gacttcgccg gtgtggtccg   3434
   ggacgacgtg accctgttca tcagcgcggt ccaggaccag gtggtgccgg acaacaccct   3494
   ggcctgggtg tgggtgcgcg gcctggacga gctgtacgcc gagtggtcgg aggtcgtgtc   3554
   cacgaacttc cgggacgcct ccgggccggc catgaccgag atcggcgagc agccgtgggg   3614
   gcgggagttc gccctgcgcg acccggccgg caactgcgtg cacttcgtgg ccgaggagca   3674
   ggactgacac gtgctacgag atttcgattc caccgccgcc ttctatgaaa ggttgggctt   3734
   cggaatcgtt ttccgggacg ccggctggat gatcctccag cgcggggatc tcatgctgga   3794
   gttcttcgcc caccccaact tgtttattgc agcttataat ggttacaaat aaagcaatag   3854
   catcacaaat ttcacaaata aagcattttt ttcactgcat tctagttgtg gtttgtccaa   3914
   actcatcaat gtatcttatc atgtctgtat accgtcgacc tctagctaga gcttggcgta   3974
   atcatggtca tagctgtttc ctgtgtgaaa ttgttatccg ctcacaattc cacacaacat   4034
   acgagccgga agcataaagt gtaaagcctg gggtgcctaa tgagtgagct aactcacatt   4094
   aattgcgttg cgctcactgc ccgctttcca gtcgggaaac ctgtcgtgcc agctgcatta   4154
   atgaatcggc caacgcgcgg ggagaggcgg tttgcgtatt gggcgctctt ccgcttcctc   4214
   gctcactgac tcgctgcgct cggtcgttcg gctgcggcga gcggtatcag ctcactcaaa   4274
   ggcggtaata cggttatcca cagaatcagg ggataacgca ggaaagaaca tgtgagcaaa   4334
   aggccagcaa aaggccagga accgtaaaaa ggccgcgttg ctggcgtttt tccataggct 4394
   ccgcccccct gacgagcatc acaaaaatcg acgctcaagt cagaggtggc gaaacccgac 4454
   aggactataa agataccagg cgtttccccc tggaagctcc ctcgtgcgct ctcctgttcc 4514
   gaccctgccg cttaccggat acctgtccgc ctttctccct tcgggaagcg tggcgctttc 4574
   tcaatgctca cgctgtaggt atctcagttc ggtgtaggtc gttcgctcca agctgggctg 4634
   tgtgcacgaa ccccccgttc agcccgaccg ctgcgcctta tccggtaact atcgtcttga 4694
   gtccaacccg gtaagacacg acttatcgcc actggcagca gccactggta acaggattag 4754
   cagagcgagg tatgtaggcg gtgctacaga gttcttgaag tggtggccta actacggcta 4814
   cactagaagg acagtatttg gtatctgcgc tctgctgaag ccagttacct tcggaaaaag 4874
   agttggtagc tcttgatccg gcaaacaaac caccgctggt agcggtggtt tttttgtttg 4934
   caagcagcag attacgcgca gaaaaaaagg atctcaagaa gatcctttga tcttttctac 4994
   ggggtctgac gctcagtgga acgaaaactc acgttaaggg attttggtca tgagattatc 5054
   aaaaaggatc ttcacctaga tccttttaaa ttaaaaatga agttttaaat caatctaaag 5114
   tatatatgag taaacttggt ctgacagtta ccaatgctta atcagtgagg cacctatctc 5174
   agcgatctgt ctatttcgtt catccatagt tgcctgactc cccgtcgtgt agataactac 5234
   gatacgggag ggcttaccat ctggccccag tgctgcaatg ataccgcgag acccacgctc 5294
   accggctcca gatttatcag caataaacca gccagccgga agggccgagc gcagaagtgg 5354
   tcctgcaact ttatccgcct ccatccagtc tattaattgt tgccgggaag ctagagtaag 5414
   tagttcgcca gttaatagtt tgcgcaacgt tgttgccatt gctacaggca tcgtggtgtc 5474
   acgctcgtcg tttggtatgg cttcattcag ctccggttcc caacgatcaa ggcgagttac 5534
   atgatccccc atgttgtgca aaaaagcggt tagctccttc ggtcctccga tcgttgtcag 5594
   aagtaagttg gccgcagtgt tatcactcat ggttatggca gcactgcata attctcttac 5654
   tgtcatgcca tccgtaagat gcttttctgt gactggtgag tactcaacca agtcattctg 5714
   agaatagtgt atgcggcgac cgagttgctc ttgcccggcg tcaatacggg ataataccgc 5774
   gccacatagc agaactttaa aagtgctcat cattggaaaa cgttcttcgg ggcgaaaact 5834
   ctcaaggatc ttaccgctgt tgagatccag ttcgatgtaa cccactcgtg cacccaactg 5894
   atcttcagca tcttttactt tcaccagcgt ttctgggtga gcaaaaacag gaaggcaaaa 5954
   tgccgcaaaa aagggaataa gggcgacacg gaaatgttga atactcatac tcttcctttt 6014
   tcaatattat tgaagcattt atcagggtta ttgtctcatg agcggataca tatttgaatg 6074
   tatttagaaa aataaacaaa taggggttcc gcgcacattt ccccgaaaag tgccacctga 6134
   cgtc 6138
<210> 4
   <211> 377
   <212> PRT
   <213> Plasmid pHA-hA4-cDNA3.1Zeo(+)
<220>
   <223> plasmid pHA-hA4-cDNA3.1Zeo(+)
<400> 4
   Met Gly Tyr Pro Tyr Asp Val Pro Asp Tyr Ala Glu Pro Ile Tyr Glu
   1               5                   10                  15
   Glu Tyr Leu Ala Asn His Gly Thr Ile Val Lys Pro Tyr Tyr Trp Leu
               20                  25                  30
   Ser Phe Ser Leu Asp Cys Ser Asn Cys Pro Tyr His Ile Arg Thr Gly
           35                  40                  45
   Glu Glu Ala Arg Val Ser Leu Thr Glu Phe Cys Gln Ile Phe Gly Phe
       50                  55                  60
   Pro Tyr Gly Thr Thr Phe Pro Gln Thr Lys His Leu Thr Phe Tyr Glu
   65                  70                  75                      80
   Leu Lys Thr Ser Ser Gly Ser Leu Val Gln Lys Gly His Ala Ser Ser
                   85                  90                  95
   Cys Thr Gly Asn Tyr Ile His Pro Glu Ser Met Leu Phe Glu Met Asn
               100                 105                 110
   Gly Tyr Leu Asp Ser Ala Ile Tyr Asn Asn Asp Ser Ile Arg His Ile
           115                 120                 125
   Ile Leu Tyr Ser Asn Asn Ser Pro Cys Asn Glu Ala Asn His Cys Cys
       130                 135                 140
   Ile Ser Lys Met Tyr Asn Phe Leu Ile Thr Tyr Pro Gly Ile Thr Leu
   145                 150                 155                     160
   Ser Ile Tyr Phe Ser Gln Leu Tyr His Thr Glu Met Asp Phe Pro Ala
                   165                 170                 175
   Ser Ala Trp Asn Arg Glu Ala Leu Arg Ser Leu Ala Ser Leu Trp Pro
               180                 185                 190
   Arg Val Val Leu Ser Pro Ile Ser Gly Gly Ile Trp His Ser Val Leu
           195                 200                 205
   His Ser Phe Ile Ser Gly Val Ser Gly Ser His Val Phe Gln Pro Ile
       210                 215                 220
   Leu Thr Gly Arg Ala Leu Ala Asp Arg His Asn Ala Tyr Glu Ile Asn
   225                 230                 235                 240
   Ala Ile Thr Gly Val Lys Pro Tyr Phe Thr Asp Val Leu Leu Gln Thr
                   245                 250                 255
   Lys Arg Asn Pro Asn Thr Lys Ala Gln Glu Ala Leu Glu Ser Tyr Pro
               260                 265                 270
   Leu Asn Asn Ala Phe Pro Gly Gln Phe Phe Gln Met Pro Ser Gly Gln
           275                 280                 285
   Leu Gln Pro Asn Leu Pro Pro Asp Leu Arg Ala Pro Val Val Phe Val
       290                 295                 300
   Leu Val Pro Leu Arg Asp Leu Pro Pro Met His Met Gly Gln Asn Pro
   305                 310                 315                 320
   Asn Lys Pro Arg Asn Ile Val Arg His Leu Asn Met Pro Gln Met Ser
                   325                 330                 335
   Phe Gln Glu Thr Lys Asp Leu Gly Arg Leu Pro Thr Gly Arg Ser Val
               340                 345                 350
   Glu Ile Val Glu Ile Thr Glu Gln Phe Ala Ser Ser Lys Glu Ala Asp
           355                 360                 365
   Glu Lys Lys Lys Lys Lys Gly Lys Lys
       370                 375
<210> 5
   <211> 6108
   <212> DNA
   <213> Plasmid phA4-cDNA3.1Zeo(+)
<220>
   <221> promoter
   <222> (232)..(819)
   <223> CMV promoter
<220>
   <221> primer_bind
   <222> (769)..(789)
   <223> CMV forward primer
<220>
   <221> primer_bind
   <222> (863)..(882)
   <223> T7 primer
<220>
   <221> promoter
   <222> (863)..(879)
   <223> T7 promoter
<220>
   <221> CDS
   <222> (941)..(2044)
   <223> hA4
<220>
   <221> primer_bind
   <222> (2115)..(2132)
   <223> BGH reverse primer
<220>
   <221> polyA_site
   <222> (2121)..(2345)
   <223> BGH pA
<220>
   <221> rep_origin
   <222> (2391)..(2819)
   <223> f1 origin
<220>
   <221> promoter
   <222> (2824)..(3194)
   <223> SV40 early promoter
<220>
   <221> promoter
   <222> (3209)..(3276)
   <223> EM7 promoter
<220>
   <221> misc_feature
   <222> (3277)..(3651)
   <223> Zeo(R)
<220>
   <221> polyA_signal
   <222> (3781)..(3911)
   <223> SV40 pA
<220>
   <221> rep_origin
   <222> (4294)..(4967)
   <223> pUC origin
<220>
   <221> misc_feature
   <222> (5112)..(5972)
   <223> Amp(R)
<220>
   <221> promoter
   <222> (5973)..(6071)
   <223> bla promoter
<400> 5
   gacggatcgg gagatctccc gatcccctat ggtcgactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata     300
   tggagttccg cgttacataa cttacggtaa atggcccgcc tggctgaccg cccaacgacc     360
   cccgcccatt gacgtcaata atgacgtatg ttcccatagt aacgccaata gggactttcc     420
   attgacgtca atgggtggac tatttacggt aaactgccca cttggcagta catcaagtgt     480
   atcatatgcc aagtacgccc cctattgacg tcaatgacgg taaatggccc gcctggcatt     540
   atgcccagta catgacctta tgggactttc ctacttggca gtacatctac gtattagtca     600
   tcgctattac catggtgatg cggttttggc agtacatcaa tgggcgtgga tagcggtttg     660
   actcacgggg atttccaagt ctccacccca ttgacgtcaa tgggagtttg ttttggcacc     720
   aaaatcaacg ggactttcca aaatgtcgta acaactccgc cccattgacg caaatgggcg     780
   gtaggcgtgt acggtgggag gtctatataa gcagagctct ctggctaact agagaaccca     840
   ctgcttactg gcttatcgaa attaatacga ctcactatag ggagacccaa gctggctagc     900
   gtttaaactt aagcttggta ccgagctcgg atccctagca atg gag ccc ata tat       955
                                               Met Glu Pro Ile Tyr
                                               1               5
   gag gag tac cta gca aat cat gga aca ata gta aaa cca tat tac tgg      1003
   Glu Glu Tyr Leu Ala Asn His Gly Thr Ile Val Lys Pro Tyr Tyr Trp
                   10                  15                  20
   cta agc ttc tct ctc gat tgc tct aat tgt cct tac cat att cga aca      1051
   Leu Ser Phe Ser Leu Asp Cys Ser Asn Cys Pro Tyr His Ile Arg Thr
               25                  30                  35
   ggt gaa gaa gca aga gtt tcc ctc aca gaa ttt tgt cag att ttt gga      1099
   Gly Glu Glu Ala Arg Val Ser Leu Thr Glu Phe Cys Gln Ile Phe Gly
           40                  45                  50
   ttc cct tat ggg aca aca ttt cct caa aca aaa cac ctc aca ttt tat      1147
   Phe Pro Tyr Gly Thr Thr Phe Pro Gln Thr Lys His Leu Thr Phe Tyr
       55                  60                  65
   gaa cta aaa act tct tct ggt agc ctg gtg caa aag ggc cat gct agc      1195
   Glu Leu Lys Thr Ser Ser Gly Ser Leu Val Gln Lys Gly His Ala Ser
   70                  75                  80                  85
   agt tgc act ggg aat tat atc cat cca gaa tca atg ctc ttt gaa atg      1243
   Ser Cys Thr Gly Asn Tyr Ile His Pro Glu Ser Met Leu Phe Glu Met
                   90                  95                  100
   aat ggt tat ctt gac tca gcc ata tac aat aat gac agc atc agg cat      1291
   Asn Gly Tyr Leu Asp Ser Ala Ile Tyr Asn Asn Asp Ser Ile Arg His
               105                 110                 115
   atc att ctg tat tcc aac aac tcc cct tgt aat gaa gct aac cac tgc      1339
   Ile Ile Leu Tyr Ser Asn Asn Ser Pro Cys Asn Glu Ala Asn His Cys
           120                 125                 130
   tgc atc agc aaa atg tat aat ttc ctg att acg tat cca ggc atc act      1387
   Cys Ile Ser Lys Met Tyr Asn Phe Leu Ile Thr Tyr Pro Gly Ile Thr
       135                 140                 145
   ctt agt att tat ttt tct cag ctc tat cat act gag atg gac ttt cct      1435
   Leu Ser Ile Tyr Phe Ser Gln Leu Tyr His Thr Glu Met Asp Phe Pro
   150                 155                 160                 165
   gcc tca gca tgg aac cgc gaa gct ctc cgg agc ctg gcc agc tta tgg      1483
   Ala Ser Ala Trp Asn Arg Glu Ala Leu Arg Ser Leu Ala Ser Leu Trp
                   170                 175                 180
   ccg cgg gtt gtt ttg agt cca ata agt ggt ggg atc tgg cat tct gtt      1531
   Pro Arg Val Val Leu Ser Pro Ile Ser Gly Gly Ile Trp His Ser Val
               185                 190                 195
   ctc cac agc ttt ata agt ggt gtc tca gga tca cat gtt ttt cag ccc      1579
   Leu His Ser Phe Ile Ser Gly Val Ser Gly Ser His Val Phe Gln Pro
           200                 205                 210
   att tta act ggg aga gca ctg gct gac agg cac aac gca tat gaa atc      1627
   Ile Leu Thr Gly Arg Ala Leu Ala Asp Arg His Asn Ala Tyr Glu Ile
       215                 220                 225
   aat gcc ata aca ggc gta aaa cct tac ttc act gat gtt ctt ctc cag      1675
   Asn Ala Ile Thr Gly Val Lys Pro Tyr Phe Thr Asp Val Leu Leu Gln
   230                 235                 240                 245
   aca aaa agg aat cca aac aca aaa gct cag gag gct tta gag agc tac      1723
   Thr Lys Arg Asn Pro Asn Thr Lys Ala Gln Glu Ala Leu Glu Ser Tyr
                   250                 255                 260
   ccc tta aac aat gcc ttt cct gga cag ttt ttt caa atg ccg agt gga     1771
   Pro Leu Asn Asn Ala Phe Pro Gly Gln Phe Phe Gln Met Pro Ser Gly
               265                 270                 275
   caa ctc caa ccc aac cta cct cca gac ctc agg gct cct gtt gtt ttt     1819
   Gln Leu Gln Pro Asn Leu Pro Pro Asp Leu Arg Ala Pro Val Val Phe
           280                 285                 290
   gtg cta gtg cct ctc agg gac tta cca cca atg cat atg ggc caa aac     1867
   Val Leu Val Pro Leu Arg Asp Leu Pro Pro Met His Met Gly Gln Asn
       295                 300                 305
   cca aat aaa ccc agg aat atc gta agg cac tta aat atg cct caa atg     1915
   Pro Asn Lys Pro Arg Asn Ile Val Arg His Leu Asn Met Pro Gln Met
   310                 315                 320                 325
   tca ttc cag gaa acc aag gac ctt gga agg ctt ccc act gga agg tca     1963
   Ser Phe Gln Glu Thr Lys Asp Leu Gly Arg Leu Pro Thr Gly Arg Ser
                   330                 335                 340
   gtg gag ata gtg gaa atc aca gaa cag ttt gca agt agc aag gag gca     2011
   Val Glu Ile Val Glu Ile Thr Glu Gln Phe Ala Ser Ser Lys Glu Ala
               345                 350                 355
   gat gaa aag aag aag aag aaa ggg aag aaa taa gaattctgca gatatccagc   2064
   Asp Glu Lys Lys Lys Lys Lys Gly Lys Lys
           360                 365
   acagtggcgg ccgctcgagt ctagagggcc cgtttaaacc cgctgatcag cctcgactgt   2124
   gccttctagt tgccagccat ctgttgtttg cccctccccc gtgccttcct tgaccctgga   2184
   aggtgccact cccactgtcc tttcctaata aaatgaggaa attgcatcgc attgtctgag   2244
   taggtgtcat tctattctgg ggggtggggt ggggcaggac agcaaggggg aggattggga   2304
   agacaatagc aggcatgctg gggatgcggt gggctctatg gcttctgagg cggaaagaac   2364
   cagctggggc tctagggggt atccccacgc gccctgtagc ggcgcattaa gcgcggcggg   2424
   tgtggtggtt acgcgcagcg tgaccgctac acttgccagc gccctagcgc ccgctccttt   2484
   cgctttcttc ccttcctttc tcgccacgtt cgccggcttt ccccgtcaag ctctaaatcg   2544
   gggcatccct ttagggttcc gatttagtgc tttacggcac ctcgacccca aaaaacttga   2604
   ttagggtgat ggttcacgta gtgggccatc gccctgatag acggtttttc gccctttgac   2664
   gttggagtcc acgttcttta atagtggact cttgttccaa actggaacaa cactcaaccc   2724
   tatctcggtc tattcttttg atttataagg gattttgggg atttcggcct attggttaaa   2784
   aaatgagctg atttaacaaa aatttaacgc gaattaattc tgtggaatgt gtgtcagtta   2844
   gggtgtggaa agtccccagg ctccccaggc aggcagaagt atgcaaagca tgcatctcaa   2904
   ttagtcagca accaggtgtg gaaagtcccc aggctcccca gcaggcagaa gtatgcaaag   2964
   catgcatctc aattagtcag caaccatagt cccgccccta actccgccca tcccgcccct 3024
   aactccgccc agttccgccc attctccgcc ccatggctga ctaatttttt ttatttatgc 3084
   agaggccgag gccgcctctg cctctgagct attccagaag tagtgaggag gcttttttgg 3144
   aggcctaggc ttttgcaaaa agctcccggg agcttgtata tccattttcg gatctgatca 3204
   gcacgtgttg acaattaatc atcggcatag tatatcggca tagtataata cgacaaggtg 3264
   aggaactaaa ccatggccaa gttgaccagt gccgttccgg tgctcaccgc gcgcgacgtc 3324
   gccggagcgg tcgagttctg gaccgaccgg ctcgggttct cccgggactt cgtggaggac 3384
   gacttcgccg gtgtggtccg ggacgacgtg accctgttca tcagcgcggt ccaggaccag 3444
   gtggtgccgg acaacaccct ggcctgggtg tgggtgcgcg gcctggacga gctgtacgcc 3504
   gagtggtcgg aggtcgtgtc cacgaacttc cgggacgcct ccgggccggc catgaccgag 3564
   atcggcgagc agccgtgggg gcgggagttc gccctgcgcg acccggccgg caactgcgtg 3624
   cacttcgtgg ccgaggagca ggactgacac gtgctacgag atttcgattc caccgccgcc 3684
   ttctatgaaa ggttgggctt cggaatcgtt ttccgggacg ccggctggat gatcctccag 3744
   cgcggggatc tcatgctgga gttcttcgcc caccccaact tgtttattgc agcttataat 3804
   ggttacaaat aaagcaatag catcacaaat ttcacaaata aagcattttt ttcactgcat 3864
   tctagttgtg gtttgtccaa actcatcaat gtatcttatc atgtctgtat accgtcgacc 3924
   tctagctaga gcttggcgta atcatggtca tagctgtttc ctgtgtgaaa ttgttatccg 3984
   ctcacaattc cacacaacat acgagccgga agcataaagt gtaaagcctg gggtgcctaa 4044
   tgagtgagct aactcacatt aattgcgttg cgctcactgc ccgctttcca gtcgggaaac 4104
   ctgtcgtgcc agctgcatta atgaatcggc caacgcgcgg ggagaggcgg tttgcgtatt 4164
   gggcgctctt ccgcttcctc gctcactgac tcgctgcgct cggtcgttcg gctgcggcga 4224
   gcggtatcag ctcactcaaa ggcggtaata cggttatcca cagaatcagg ggataacgca 4284
   ggaaagaaca tgtgagcaaa aggccagcaa aaggccagga accgtaaaaa ggccgcgttg 4344
   ctggcgtttt tccataggct ccgcccccct gacgagcatc acaaaaatcg acgctcaagt 4404
   cagaggtggc gaaacccgac aggactataa agataccagg cgtttccccc tggaagctcc 4464
   ctcgtgcgct ctcctgttcc gaccctgccg cttaccggat acctgtccgc ctttctccct 4524
   tcgggaagcg tggcgctttc tcaatgctca cgctgtaggt atctcagttc ggtgtaggtc 4584
   gttcgctcca agctgggctg tgtgcacgaa ccccccgttc agcccgaccg ctgcgcctta 4644
   tccggtaact atcgtcttga gtccaacccg gtaagacacg acttatcgcc actggcagca 4704
   gccactggta acaggattag cagagcgagg tatgtaggcg gtgctacaga gttcttgaag   4764
   tggtggccta actacggcta cactagaagg acagtatttg gtatctgcgc tctgctgaag   4824
   ccagttacct tcggaaaaag agttggtagc tcttgatccg gcaaacaaac caccgctggt   4884
   agcggtggtt tttttgtttg caagcagcag attacgcgca gaaaaaaagg atctcaagaa   4944
   gatcctttga tcttttctac ggggtctgac gctcagtgga acgaaaactc acgttaaggg   5004
   attttggtca tgagattatc aaaaaggatc ttcacctaga tccttttaaa ttaaaaatga   5064
   agttttaaat caatctaaag tatatatgag taaacttggt ctgacagtta ccaatgctta   5124
   atcagtgagg cacctatctc agcgatctgt ctatttcgtt catccatagt tgcctgactc   5184
   cccgtcgtgt agataactac gatacgggag ggcttaccat ctggccccag tgctgcaatg   5244
   ataccgcgag acccacgctc accggctcca gatttatcag caataaacca gccagccgga   5304
   agggccgagc gcagaagtgg tcctgcaact ttatccgcct ccatccagtc tattaattgt   5364
   tgccgggaag ctagagtaag tagttcgcca gttaatagtt tgcgcaacgt tgttgccatt   5424
   gctacaggca tcgtggtgtc acgctcgtcg tttggtatgg cttcattcag ctccggttcc   5484
   caacgatcaa ggcgagttac atgatccccc atgttgtgca aaaaagcggt tagctccttc   5544
   ggtcctccga tcgttgtcag aagtaagttg gccgcagtgt tatcactcat ggttatggca   5604
   gcactgcata attctcttac tgtcatgcca tccgtaagat gcttttctgt gactggtgag   5664
   tactcaacca agtcattctg agaatagtgt atgcggcgac cgagttgctc ttgcccggcg   5724
   tcaatacggg ataataccgc gccacatagc agaactttaa aagtgctcat cattggaaaa   5784
   cgttcttcgg ggcgaaaact ctcaaggatc ttaccgctgt tgagatccag ttcgatgtaa   5844
   cccactcgtg cacccaactg atcttcagca tcttttactt tcaccagcgt ttctgggtga   5904
   gcaaaaacag gaaggcaaaa tgccgcaaaa aagggaataa gggcgacacg gaaatgttga   5964
   atactcatac tcttcctttt tcaatattat tgaagcattt atcagggtta ttgtctcatg   6024
   agcggataca tatttgaatg tatttagaaa aataaacaaa taggggttcc gcgcacattt   6084
   ccccgaaaag tgccacctga cgtc                                          6108
<210> 6
   <211> 367
   <212> PRT
   <213> Plasmid phA4-cDNA3.1Zeo(+)
<220>
   <223> Plasmid phA4-cDNA3.1Zeo(+)
<400> 6
   Met Glu Pro Ile Tyr Glu Glu Tyr Leu Ala Asn His Gly Thr Ile Val
   1               5                   10                  15
   Lys Pro Tyr Tyr Trp Leu Ser Phe Ser Leu Asp Cys Ser Asn Cys Pro
               20                  25                  30
   Tyr His Ile Arg Thr Gly Glu Glu Ala Arg Val Ser Leu Thr Glu Phe
           35                  40                  45
   Cys Gln Ile Phe Gly Phe Pro Tyr Gly Thr Thr Phe Pro Gln Thr Lys
       50                  55                  60
   His Leu Thr Phe Tyr Glu Leu Lys Thr Ser Ser Gly Ser Leu Val Gln
   65                  70                  75                  80
   Lys Gly His Ala Ser Ser Cys Thr Gly Asn Tyr Ile His Pro Glu Ser
                   85                  90                  95
   Met Leu Phe Glu Met Asn Gly Tyr Leu Asp Ser Ala Ile Tyr Asn Asn
               100                 105                 110
   Asp Ser Ile Arg His Ile Ile Leu Tyr Ser Asn Asn Ser Pro Cys Asn
           115                 120                 125
   Glu Ala Asn His Cys Cys Ile Ser Lys Met Tyr Asn Phe Leu Ile Thr
       130                 135                 140
   Tyr Pro Gly Ile Thr Leu Ser Ile Tyr Phe Ser Gln Leu Tyr His Thr
   145                 150                 155                 160
   Glu Met Asp Phe Pro Ala Ser Ala Trp Asn Arg Glu Ala Leu Arg Ser
                   165                 170                 175
   Leu Ala Ser Leu Trp Pro Arg Val Val Leu Ser Pro Ile Ser Gly Gly
               180                 185                 190
   Ile Trp His Ser Val Leu His Ser Phe Ile Ser Gly Val Ser Gly Ser
           195                 200                 205
   His Val Phe Gln Pro Ile Leu Thr Gly Arg Ala Leu Ala Asp Arg His
       210                 215                 220
   Asn Ala Tyr Glu Ile Asn Ala Ile Thr Gly Val Lys Pro Tyr Phe Thr
   225 230 235 240
   Asp Val Leu Leu Gln Thr Lys Arg Asn Pro Asn Thr Lys Ala Gln Glu
   245 250 255
   Ala Leu Glu Ser Tyr Pro Leu Asn Asn Ala Phe Pro Gly Gln Phe Phe
   260 265 270
   Gln Met Pro Ser Gly Gln Leu Gln Pro Asn Leu Pro Pro Asp Leu Arg
   275 280 285
   Ala Pro Val Val Phe Val Leu Val Pro Leu Arg Asp Leu Pro Pro Met
   290 295 300
   His Met Gly Gln Asn Pro Asn Lys Pro Arg Asn Ile Val Arg His Leu
   305 310 315 320
   Asn Met Pro Gln Met Ser Phe Gln Glu Thr Lys Asp Leu Gly Arg Leu
   325 330 335
   Pro Thr Gly Arg Ser Val Glu Ile Val Glu Ile Thr Glu Gln Phe Ala
   340 345 350
   Ser Ser Lys Glu Ala Asp Glu Lys Lys Lys Lys Lys Gly Lys Lys
   355 360 365
<210> 7
   <211> 32
   <212> DNA
   <213> Artificial
<220>
   <223> Forward primer
<400> 7
   caggcggtac cagcctggag acaaattgat gg 32
<210> 8
   <211> 57
   <212> DNA
   <213> Artificial
<220>
   <223> Reverse primer
<400> 8
   gacttggatc ccctttcttc cctttcttct tcttcttttc atctgcctcc ttgctac 57
<210> 9
   <211> 68
   <212> DNA
   <213> Artificial
<220>
   <223> Forward primer
<400> 9
   cggatcccta gcaatgggat atccatacga tgttccagat tacgctgagc ccatatatga 60
   ggagtacc 68
<210> 10
   <211> 32
   <212> DNA
   <213> Artificial
<220>
   <223> Reverse primer
<400> 10
   gaattcttta tttcttccct ttcttcttct tc 32
<210> 11
   <211> 29
   <212> DNA
   <213> Artificial
<220>
   <223> Forward primer
<400> 11
   cggatcccta gcaatggagc ccatatatg 29
<210> 12
   <211> 32
   <212> DNA
   <213> Artificial
<220>
   <223> Reverse primer
<400> 12
   gaattcttta tttcttccct ttcttcttct tc 32

## Description

### Technical Field

The invention relates to the modulation of the release of retroviruses or viral particles thereof from cells by human APOBEC4 or a modified human APOBEC4 and various uses thereof.

### Background Art

The apolipoprotein B mRNA-editing enzyme catalytic polypeptide-like 4 (APOBEC4) family is a member of the vertebrate specific AID/APOBEC superfamily of RNA/DNA editing cytidine deaminases **(**ROGOZIN, IB, et al. APOBEC4, a new member of the AID/APOBEC family of polynucleotide (deoxy)cytidine deaminases predicted by computational analysis. Cell Cycle. 2005, vol.4, no.9, p.1281-5.) The AID/APOBEC protein superfamily contains five subfamilies (AID, APOBEC1, APOBEC2, APOBEC3 and APOBEC4) and includes several members with the capability of deaminating cytosine to uracil in single-stranded polynucleotides, while fulfilling diverse physiological functions **(**CONTICELLO, SG, et al. Evolution of theAID/APOBEC family of polynucleotide (deoxy)cytidine deaminases. Mol Biol Evol. 2005, vol.22, no.367, p.77), amongst which is the inhibition of retroviruses by nucleic acid editing dependent and independet mechanisms **(**HOLMES, RK, et al. APOBEC-mediated viral restriction: not simply editing? Trends Biochem Sci. 2005, vol.32, no.3, p.118-128). A method to inactivate APOBEC enzymes by means of the foamy virus Bet has been described **(**WO 2005/117947**),** and WO 2006/065377 discloses a method for the identification of agents that reduce the level of active APOBEC3C in a cell.

In mammals, expression of APOBEC4 is upregulated in testis which suggests the possibility that it is an editing enzyme for mRNAs involved in spermatogenesis. However, the exact physiological function(s) of APOBEC4 are still unknown. The nucleic acid and amino acid sequence for APOBEC4 is known and available for various species, *inter alia,* human (GenBank accession number NM_203454, Gl 44888831), mouse (GenBank accession number NC_000067, Gl 38073497), or rat (GenBank accession number NM_001017492, Gl 62945336). APOBEC4 is a protein of about 370 amino acids and 41000 Da (human APOBEC4: 367 amino acids; 41581 Da) and comprises a zinc finger domain and, in most species, a C-terminal poly-lysine tail.

### Disclosure of Invention

The invention relates to subject matter as defined in the appended claims. The present invention is based on the discovery that expression of the APOBEC4 protein or a modified APOBEC4 protein in a cell producing a retrovirus leads to an increased or decreased production of retroviruses or viral particles thereof from said cell. Specifically, stabilization of the protein by modification of the N-terminus, *inter alia,* by addition of an amino acid sequence, for example, the V5 tag, the c-myc tag or the HA tag, leads to an increased production of retroviruses or viral particles thereof from said cell. Modification of the C-terminus, *inter alia,* by addition of at least one V5 tag, c-myc tag or HA tag leads to a decreased production of retroviruses or viral particles thereof from said cell. It is further disclosed that the C-terminal poly-lysine tail of APOBEC4 can be modified by deletion or modification by mutation.

A first aspect of the invention is a modified human APOBEC4 protein characterized in that the N-terminus is modified to stabilize the APOBEC4 protein or the C-terminus is modified. The modification of the N-terminus comprises addition of at least one amino acid sequence selected from the group consisting of the V5 tag, the c-myc tag and the HA tag or an amino acid sequence homologous thereto. The at least one amino acid sequence may be HA or at least 90% identical thereto. The modification of the C-terminus comprises addition of at least one amino acid sequence selected from the group consisting of the V5 tag, the c-myc tag and the HA tag or an amino acid sequence homologous thereto. More preferably, the at least one amino acid sequence is HA, 3 HA or at least 90%identical thereto.

A further aspect of the invention is a cell expressing the C- or N-terminally modified APOBEC4 protein of the present invention. Preferably, the cell further produces a retrovirus. More preferably, the retrovirus or lentivirus is selected from the group consisting of HIV, HIV-1, HIV-2, SIV, SIVagm, SIVmac, FIV, EIAV, spuma virus (foamy virus) and MLV. Even more preferably, said retrovirus or lentivirus is a replication deficient virus. In a further preferred embodiment the cell is selected from the group consisting of a human cell, a HeLa cell, a 293T cell, a stem cell, a T-helper cell, a macrophage or progenitors or cell lines thereof.

Another aspect of the present invention is a nucleic acid comprising the nucleic acid sequence coding for the modified APOBEC4 protein of the present invention or the complement thereof. In a preferred embodiment the nucleic acid comprises a nucleic acid coding for the APOBEC4 protein of the present invention or complement thereof, operably linked to a promoter. In a more preferred embodiment the nucleic acid is a vector for gene therapy. In a further more preferred embodiment the promoter is the CMV promoter.

Another aspect of the present invention is the use of the nucleic acid sequence or cell of the present invention for the production of a medicament. In a preferred embodiment the nucleic acid is a vector for gene therapy and/or a nucleic acid comprising the nucleic acid coding for the modified APOBEC4 protein of the present invention operably linked to a promoter, preferably the CMV promoter. In a further preferred embodiment the cell expresses the modified APOBEC4 protein of the present invention. In a further preferred embodiment the cell expresses the modified APOBEC4 protein of the present invention and furthermore produces a retrovirus or virus particles thereof, preferably a replication deficient retrovirus, more preferably a replication deficient virus selected from the group consisting of HIV, SIV, MLV, FIV, EIAV, HIV-1, HIV-2, SIVagm, SIVmac, spuma virus or virus particles thereof. In a further aspect the medicament is directed to the treatment of or vaccination against HIV, SIV, MLV, FIV, EIAV, HIV-1, HIV-2, spuma virus, SIVagm and SIVmac.

Another aspect of the invention is the use of a viral particle of the invention for the preparation of a medicament, preferably for the preparation of a medicament for the treatment of viral infections. In a preferred embodiment, the viral particles comprise a C-terminally modified APOBEC4 protein of the invention, preferably the APOBEC4-HA or the APOBEC4-3xHA protein. In another preferred embodiment the viral particles are based on a virus selected from the group consisting of HIV, SIV, MLV, FIV, EIAV, HIV-1, HIV-2, SIVagm, SIVmac, spuma virus, preferably HIV-1. In a further preferred embodiment, the viral particles are pseudotyped viral particles. In another embodiment of the present invention the viral particle encodes sh/siRNA which targets the endogenous APOBEC4 and further encodes a codon optimized therapeutic APOBEC4, for example APOBEC-3xHA or APOBEC-HA, which is not affected by the sh/siRNA molecules.

Another aspect of the present invention is a vaccine comprising the cell of the present invention and an acceptable pharmaceutical carrier. In a preferred embodiment, the cell expresses the modified APOBEC4 protein of the present invention, wherein the APOBEC4 protein is modified at the N-terminus to stabilize the APOBEC4 protein and further produces a retrovirus or virus particles thereof, preferably a replication deficient retrovirus or lentivirus. In a further preferred embodiment the cell is a stem cell or an autologous cell, a T-helper cell or a macrophage.

Another aspect of the present invention is a method for decreasing the production of retroviruses or viral particles thereof of a cell producing a retrovirus or viral particles thereof, the method comprising expressing a modified APOBEC4 protein of the present invention in said cell, wherein the C-terminus of the APOBEC4 protein is modified to decrease or inhibit binding to the C-terminal poly-lysine tail of APOBEC4. In a preferred embodiment, the production of retroviruses or viral particles thereof is decreased by at least 20%, 30%, 40%, 50%, 60%, 70%, 80%, 85%, 90%, 95%, 97% or 99%.

A further aspect of the present invention is a method for producing retroviral particles, the method comprising cultivating a cell according to the present invention, wherein the cell produces a replication deficient retrovirus or virus particles of a retrovirus and furthermore expresses a modified APOBEC4 protein of the present invention, wherein the N-terminus of the APOBEC4 protein is modified to stabilize the APOBEC4 protein. In a preferred embodiment, the N-terminus of the APOBEC4 protein is modified by addition of a HA tag. In a further preferred embodiment the cell is a 293T cell or a HeLa cell co-transfected with an HA-APOBEC4 fusion construct and a HIV-1 expression plasmid.

A further aspect of the present invention is a method for screening for antiviral compounds, the method comprising the steps of contacting a cell expressing a human APOBEC4 protein with a test compound, determining whether the test compound affects APOBEC4 transcription and/or expression of said cell. In a preferred embodiment the APOBEC4 transcription and/or expression of a first cell in the presence of the test compound is compared to the APOBEC4 transcription and/or expression of a second cell not contacted with the test compound, wherein a decrease in APOBEC4 transcription and/or expression of the first cell indicates that the test compound is a potential antiviral compound.

In a preferred embodiment, HeLa or 293T cells are used in the screening method. In a further preferred embodiment the amount of APOBEC4 transcript and/or APOBEC4 protein is determined by RT-PCR, Northern blotting and western blotting, respectively.

A further aspect of the invention is a method for screening for antiviral compounds, the method comprising the steps of contacting a cell expressing a retrovirus and human APOBEC4 with a test compound and determining the titer of the retrovirus in the presence and the absence of the test compound, wherein a decrease in the titer indicates that the candidate compound interferes with the assembly of the retrovirus by interaction with the APOBEC4 protein and thus is a potential antiretroviral drug.

Further disclosed are molecules - and pharmaceutics based on such molecules - directed to and interfering with the APOBEC4 mRNA. Preferably said molecules are siRNA or shRNA molecules directed against APOBEC4 mRNA. Preferably, such molecules are used for the manufacture of a medicament. Even more preferably, said molecules are used for the preparation of a medicament for the treatment of viral infections.

Additional embodiments of the present invention are described in the claims and the detailed description of the invention.

### Brief Description of Figures in the Drawings

Figure 1 is a schematic structural overview over the human APOBEC4 protein and consequences of modification of the C-terminus, exemplarily demonstrated by an HA-tag.

Figure 2 depicts the vector maps of plasmids phA4-3xHA (Fig. 2A), phHA-A4cDNA3.1Zeo(+) (Fig 2B) and phA4cDNA3.1Zeo(+) (Fig. 2C), respectively.

Figure 3 shows the results of the western blotting of 293T cells co-transfected with HIV-1 NL4-3 and an expression plasmid coding for an unmodified APOBEC4, HA-APOBEC4 and APOBEC4-3xHA, respectively.

Figure 4 shows the results of the western blotting of T293 cells co-transfected an expression plasmid encoding the reporter virus HIV-1 NL-luc R-E- and the expression plasmid coding for the unmodified APOBEC4, HA-APOBEC4 and APOBEC4-3xHA, respectively.

Figure 5 shows the results of the reporter virus assays, wherein co-transfection of the cells with the reporter virus HIV-1 NL-luc R-E- and the expression plasmid coding for the unmodified APOBEC4, HA-APOBEC4 and APOBEC4-3HAare shown.

### Detailed Description

### Definitions

"**APOBEC4**", as used in the present invention refers to the apolipoprotein B mRNA-editing enzyme catalytic polypeptide-like 4 protein or gene coding for said protein. The modified APOBEC4 proteins of the present invention that contain additional amino acid sequences, for example a tag like the HA tag, are designated as HA-APOBEC4 if the tag is attached to the N-terminus of the APOBEC4 protein and APOBEC4-HA if the tag is attached to the C-terminus of the APOBEC4 protein. Thus, V5-APOBEC4 and APOBE4-V5 designate a fusion protein, wherein the V5 tag is fused to the N- and C-terminus of the APOBEC4 protein, respectively. As a further example, APOBEC4-3xHA designates a fusion protein of APOBEC4 and three HA tags sequentially fused to the C-terminus of APOBEC4. Nucleic acids or plasmids are designated in the same manner.

The "**viruses**" of the present invention encompass all retroviruses, preferably lentiviruses, more preferably HIV, SIV, FIV, EIAV, MLV, spuma virus, even more preferably HIV-1, HIV-2, SIVagm, SIVmac, and most preferably HIV-1. Furthermore, mutated and/or truncated viruses derived from the above retroviruses are encompassed by the present invention. A subset of such mutated/truncated retroviruses are the so called "**vector particles"** or "**viral particles**", which refer to replication deficient retroviruses. Thus, the retroviral vector particles of the present invention are retroviral vectors that may contain an RNA which, upon infection of a host or target cell, is reverse-transcribed and inserted into the genome of said cell. This RNA may contain a heterologous gene, for example the luciferase reporter gene of *Photinus pyralis* as in the NL-luc R-E- reporter virus described *infra.* However, these vector particles only contain an incomplete genome of the lentivirus from which they are derived. In general, the RNA molecule of the vector particle does not comprise the genetic information of the *gag, env,* and/or pol genes itself, which is a known minimal requirement for successful replication of a retrovirus. As a result, the vector particles are replication deficient, i.e. they are not able to transduce a host or target cell with the genetic information required for their own replication.

A vector particle thus comprises a minimum of the Gag, Pol, and Env proteins and an RNA molecule (which can be an expression vector). The RNA molecule is derived from the genome of the retrovirus but does not comprise at least one of the *gag, env,* or pol genes itself. However, to produce a safe and efficient vector particle, in general all three of said genes are lacking, as well as any other nonessential genes. In case of HIV-1, such unnecessary genes of the HIV-1 genome that can be deleted from the RNA molecule include *tat, vif, vpr, vpu* and nef.

The RNA molecule may still comprise all elements, for example, the psi element and LTRs, of the retroviral genome which are required for an effective packaging of the RNA into the resulting vector particles. In the case of an expression vector, the RNA molecule preferably comprises a further gene (usually heterologous) that is under the control of a suitable promoter, for example, the CMV promoter, and is thus expressed upon integration of the gene into the genome of the host or target cell.

Therefore, one example of an RNA molecule that may be used to generate a retroviral vector particle is based on the HIV-1 genome and comprises the LTRs, the psi element and the CMV promoter followed by the gene to be transduced, for example, a reporter gene such as the gene for a GFP protein or the *luciferase* reporter gene of *Photinus pyralis.* On expression of such reporter genes, the titers or the amount of the generated vector particles can conveniently be determined by standard assays. The *gag, env, pol, tat, vif, vpr, vpu* and/or nef genes of HIV-1 are removed or expression of their gene products is prevented by, for example, frame shift mutation(s).

Another example for a heterologous gene comprised by the RNA molecule is the gene coding for the (modified) APOBEC4 protein of the present invention. Thus, the present invention provides vector particles that are based on and display the tropism of a specific retrovirus, for example HIV-1, and for which treatment is sought and further comprise a gene coding for a modified APOBEC4 protein of the present invention that decreases production of the retrovirus. Such vector particles will transfect the same cells, possibly infected by HIV-1, and subsequently decrease production of HIV-1 by said cells.

The minimal requirements for a lentivirus vector based on HIV-1 have been described by KIM, VN, et al. Minimal requirement for a lentivirus vector based on human immunodeficiency virus type 1. J Virol. 1998, vol.72, no.1, p.811-6.

The above-described RNA molecule together with the *gag, pol* and *env* proteins, that may be provided *in trans* by the packaging cell line, are then assembled into the vector particles or vector particles, which will be able to efficiently infect their target or host cells, reverse-transcribe the RNA molecule that may comprise a heterologous gene under the control of a promoter, for example the CMV promoter, and integrate said genetic information into the genome of the target/host cells. However, as the genetic information for the *gag, pol* and/or *env* proteins is not present on the transduced RNA molecule, the vector particles or vector particles will be replication deficient, i.e. no new generation of said vector particles will thus be generated by the transduced cell.

The term "**stabilization**", as used in the present invention refers to an inhibition or decrease of protein degradation or an inhibited or decreased transport of the protein from, for example, the cytosol into another cellular compartment or out of the cell, thus leading to an increased availability of the protein within the cell, for example, in the cytosol. The skilled person is aware of various ways to achieve stabilization of a protein, *inter alia,* by modification of the protein through amino acid substitutions, additions or deletions. A preferred modification to achieve stabilization is the N-terminal addition of one or more amino acid sequences selected from the group consisting of the V5 tag, the c-myc tag and the HA tag or an amino acid sequence homologous thereto.

The person skilled in the art will readily be able to introduce mutations as, for example, additions and deletions, into a given nucleic acid or amino acid sequence. Such known methodologies are, for example, disclosed in Sambrook *et al.* (1989).

The invention particularly includes nucleic acids sequences or homologs thereof, or unique fragments thereof. In the present invention, the sequence of a nucleic acid molecule that encodes a resulting protein is considered homologous to a second nucleic acid molecule if the nucleotide sequence of the first nucleic acid molecule is at least about 70% identical, preferably at least about 80% identical, and more preferably at least about 85%, 90%, 95% or 99% identical to the sequence of the second nucleic acid molecule. Alternatively, homology between two nucleic acid sequences may be readily determined using the known BLASTN algorithm (ALTSCHUL, SF, et al. Basic local alignment search tool. J Mol Biol. 1990, viol.215, no.3, p.403-10) with default settings. As a further example, another known test for ascertaining the homology of two nucleic acid sequences is whether they hybridize under normal hybridization conditions, preferably under stringent hybridization conditions.

Using the nucleic acid sequences disclosed herein, the skilled person can further design nucleic acid structures having particularly desired functions in various types of applications. For example, the artisan can construct oligonucleotides or polynucleotides for use as primers in nucleic acid amplification procedures, such as the polymerase chain reaction (PCR), ligase chain reaction (LCR), Repair Chain Reaction (RCR), PCR oligonucleotide ligation assay (PCR-OLA), and the like. Oligonucleotides useful as probes in hybridization studies, such as *in situ* hybridization, can also be constructed. Numerous methods for labeling such probes with radioisotopes, fluorescent tags, enzymes, and binding moieties (e.g., biotin) are known, thus the probes of the present invention can be adapted for detectability in a straightforward manner.

The protein of the present invention further includes functional homologs. A protein is considered a functional homolog of another protein for a particular function, if the homolog has a similar function as the original protein. The homolog can be, for example, a fragment of the protein, or a substitution, addition, or deletion mutant of the protein.

Determining whether two amino acid sequences are substantially homologous is typically based on FASTA searches in accordance with Pearson *et al.* PERASON, WR, et al. Improved tools for biological sequence comparison. Proc Natl Acad Sci U S A. 1988, vol.85, no.8, p.2444-8. For example, the amino acid sequence of a first protein is considered to be homologous to that of a second protein if the amino acid sequence of the first protein shares at least about 70% amino acid sequence identity, preferably at least about 80% identity, and more preferably at least about 85%, 90%, 95%, 97%, 99% or 99.5%, most preferably at least 99% identity, with the sequence of the second protein.

The term "**pseudotyped**", as used in the present invention, refers to a vector particle bearing envelope glycoproteins derived from other viruses having envelopes. The host range of the retroviruses or lentiviruses of the present invention can thus be expanded or altered depending on the type of cell surface receptor used by the glycoprotein.

The *gag, pol* and *env* proteins needed to assemble the vector particle are provided *in trans* by means of a packaging cell line, for example, HEK-293T. This is usually accomplished by transfection of the packaging cell line with one or more plasmids containing the *gag, pol* and *env* genes. For the generation of pseudotyped vectors, the *env* gene, originally derived from the same retrovirus as the *gag* and *pol* genes and as the RNA molecule or expression vector, is exchanged for the envelope protein(s) of a different enveloped virus. As an example, the G protein of VSV is used.

Thus, an exemplary pseudotyped vector particle based on the HIV-1 retrovirus comprises the (1) HIV-1 Gag and Pol proteins, (2) an RNA molecule derived from the HIV-1 genome that may be used to generate a retroviral vector particle based on the HIV-1 genome lacking the *gag, env, pol, tat, vif, vpr, vpu* and *nef* genes, but still comprising the LTRs, the psi element and a CMV promoter followed by the gene to be transduced, for example, a gene for the GFP protein, and (3) the G protein of VSV. The resulting pseudotyped vector particle will thus display the tropism of VSV.

A further example is the reporter virus NL-luc R-E- that is derived from the HIV-1 variant NL4-3 and features a deletion of the vpr, *env and nef genes.* Furthermore, NL-luc R-E- carries the *luciferase* reporter gene of *Photinus pyralis* and is pseudotyped with the VSV-G protein.

A virus or vector particle "**derived from**", for example, HIV-1, as used in the present invention, refers to a virus or vector particle in which the genetic information for the RNA and/or the Gag and Pol proteins comprised by the virus or vector particle originally stems from the original virus, in the above case, HIV-1. As described above, the original retroviral genome can comprise mutations, such as deletions, frame shift mutations and insertions.

The term "**tag**" as used in the present invention, refers to a short amino acid sequence which serves as an epitope for immunological detection of protein of interest. Tags used in the present invention are HA, V5 and c-myc.

### Taxonomy

Viruses of the taxonomic family of the *Retroviridae* share, as a unifying feature, a positive stranded ssRNA genome (herein "ss(+)RNA"). During infection, said ss(+)RNA can not directly be used as a template (mRNA), thus the genetic information of this virus is first reverse-transcribed from RNA into DNA by a reverse transcriptase provided by the virus and subsequently integrated into the host genome. Thus, the term "retro" refers to the activity of reverse transcriptase and the transfer of genetic information from RNA to DNA. Presently, more than 570 individual viruses of the family have been identified.

One sub-family of *Retroviridae* comprises the *Orthoretroviridae* viruses, including the genera of the alpha-retroviruses with, for example, the Avian leukosis virus (ALV) species the gamma-retroviruses with, for example, the Murine leukemia virus (MLV) species or the Feline leukemia virus (FLV) or the delta-retroviruses with, for example, the Bovine leukemia virus (BLV) species or the Human T-lymphotropic virus (HTLV).

One additional genus of the *Orthoretroviridae,* known as the lentiviruses, has recently attracted much interest largely because of a well-known member, the Human immunodeficiency virus (HIV). However, the genus of lentiviruses comprises numerous other viruses. In addition to the HIV-1 or HIV-2 species, the Bovine immunodeficiency virus (BIV), the Feline immunodeficiency virus (FIV) and the Simian immunodeficiency virus (SIV) are all examples of frequent and extensive research efforts. A general overview of the retroviruses and lentiviruses, respectively can be found, for example, in Vigna *et al.* and Palu *et al.* **(**VIGNA, E, et al. Lentiviral vectors: excellent tools for experimental gene transfer and promising candidates for gene therapy. J Gene Med. 2000, vol.2, no.5, p.308-16. PALU, G, et al. Progress with retroviral gene vectors. Rev Med Virol. 2000, viol.10, no.3, p.185-202).

### Structural and Genetic Features of Retroviral Genomes

The genomes of known replication-competent retroviruses typically comprise the four coding domains *(gag, pro, pol, env),* wherein *gag* encodes a polypeptide (herein "Gag") wherein the cleavage products comprise the major structural proteins of the virus core including Matrix (MA), capsid (CA), and nucleocapsid (NC). Pro encodes part of the polyprotein (Gag-Pro or Gag-Pro-Pol) wherein the cleavage products typically include protease (PR) and occasionally dUTPase (DU). *Pol* encodes part of the polyprotein (Gag-Pro-Pol), wherein the cleavage products typically include reverse transcriptase (RT) and integrase (IN) and, in some lentiviruses, dUTPase (DU). In spumaviruses, *pol* is expressed via a spliced mRNA as Pol-Pol polyprotein. Env encodes a polyprotein (Env) whose cleavage products SU (surface) and TM (transmembrane) comprise the structural proteins of the viral envelope.

Furthermore, retroviruses typically contain two long terminal repeats (LTRs) and a region of several hundred (∼300-1800) base pairs composed of U3-R-U5 (5to 3) which are located at both ends of the unintegrated and integrated (proviral) genome. Additionally, a psi element is generally present within the retroviral genome. The psi element is a cis-acting signal, located near the 5' end of the genome and designates a packaging signal, which is of importance during virus assembly and leads to the incorporation of the viral RNA into the viral core. More complex retroviruses typically comprise additional regulatory genes. In case of HIV-2, these regulatory genes include *ref, tat, vif, nef, vpr* and *vpx.*

### Overview of the Function of APOBEC4 in Virus Production

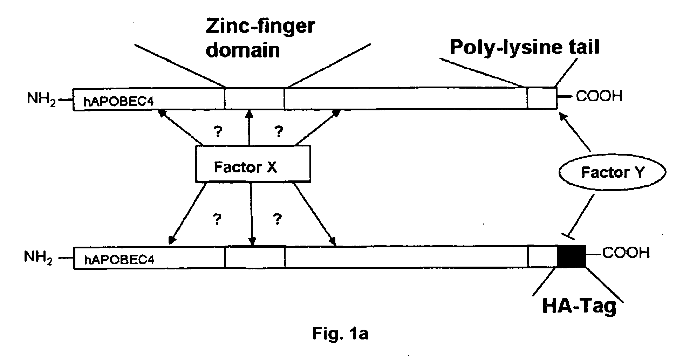

APOBEC4 is a eukaryotic protein and expressed primarily in mammalian testis which suggesting that it is an editing enzyme for mRNAs involved in spermatogenesis. As a non-limiting working hypothesis (see Figure 1a), during viral replication a complex comprising APOBEC4 and at least two further and yet unknown factors (factor X and factor Y) is recruited and used by the replication machinery of the virus. As depicted in Figure 1a, APOBEC4 comprises a zinc-finger domain (hpesmlfemngyldsaiynndsirhiilysnnspcneanhcc, aa 93-134) and a C-terminal poly-lysine tail (kkkkkgkk, aa 360-367), wherein the poly-lysine tail enables binding of factor Y, while factor X presumably binds to the zinc-finger domain. Furthermore, a surplus within the cell of the two factors, X and Y, compared to endogenous APOBEC4 is assumed.

The present invention is based on the surprising experimental finding that expression of the APOBEC4 protein in a cell producing a retrovirus leads to an increased or decreased production of retroviruses from said cell. Furthermore, this pro-viral effect directly correlated with the amount of plasmid DNA coding for the APOBEC4 protein used for the co-transfection and thus with the expressed APOBEC4 protein. This correlating pro-viral effect of APOBEC4 expression can be explained with an increased formation of the APOBEC4 complex recruited by the virus: Since there is a surplus of factor X and Y, the amount of APOBEC4 complexed with factor X and factor Y will only depend on the amount of APOBEC4 expressed by the cell (and thus depend on the amount of plasmid DNA used during transformation). An increase in virus production up to 10-fold was achieved using HA-APOBEC4 compared to the negative control (293T transfected only with provirus).

Proteins are constantly degraded and re-synthesized by the cell (a process known as protein turnover) or transported or translocated into various cellular compartments, for example, from the cytosol into the mitochondria. Consequently, if protein turnover is shifted, i.e. the protein degradation is prevented or slowed down, or transport of the protein from the cytosol into another cellular compartment is inhibited or slowed down, the protein will accumulate in the cytosol of the cell.

Surprisingly, the inventors discovered that stabilization/modification of the APOBEC4 protein by modification of the N-terminus, *inter alia,* by addition of an amino acid sequence, for example, the V5 tag, the c-myc tag or the HA tag, and expression of such a protein in a cell producing a retrovirus or viral particles thereof leads to an augmented increased production of retroviruses or viral particles by said cell compared to expression of unmodified APOBEC4 within that cell. The amount of viruses that were detectable by reverse transcriptase assays or western blotting within about 2 days after co-transfection in the supernatant of cells showed a 5-10 fold increase.

Another aspect of the present invention is based on the surprising discovery that modification of the C-terminus of the APOBEC4 protein, *inter alia,* by addition of at least one V5 tag, c-myc tag or HA tag, and subsequent expression of such a modified APOBEC4 protein leads to a drastically decreased production (up to 95% or more) of retroviruses or viral particles thereof by said cell. Furthermore, this antiviral-viral effect directly correlated with the amount of plasmid DNA coding for the modified APOBEC4 protein used for the co-transfection and thus with the modified APOBEC4 protein expressed by the cell. This correlating anti-viral effect of the present invention can be explained by competition of expressed modified APOBEC4 with endogenous APOBEC4 for the factor X. The APOBEC4 protein modified at the C-terminus will bind factor X but will be unable to bind factor Y in order to form a functional APOBEC4 complexed with factor X and factor Y that is required by the virus for replication. Consequently, as the modified APOBEC4 competes for factor X, less of said factor will be available to the endogenous APOBEC4 and thus less functional APOBEC4 complex will be formed, leading to the observed decrease in virus production by the cell, i.e. the anti-viral effect.

### APOBEC4 Proteins and Constructs

It is disclosed that APOBEC4 proteins of various species, mammals and humans exist. The present invention makes use of human APOBEC4. The preferred APOBEC4 proteins encompassed by the invention are APOBEC4 proteins modified according to the teachings disclosed herein.

Generally, modified APOBEC4 proteins are encompassed by the present invention that are stabilized, preferably by modification of, for example, the N-terminus, *inter alia,* by addition of an amino acid sequence, for example, the V5 tag, the c-myc tag or, preferably, the HA tag. Furthermore, amino acid sequences that are homologous to the V5 tag, the c-myc tag or, preferably, the HA tag, preferably, at least 90% identical thereto, are also explicitly mentioned as amino acid sequences that may be used to modify the APOBEC4 protein according to the present invention. Furthermore, APOBEC4 proteins that are modified to inhibit or decrease binding of at least one factor required to form the APOBEC4 complex that is recruited during viral replication are disclosed. The skilled person is aware of a multitude of means to modify a protein to inhibit or decrease binding of factors thereto. Preferred APOBEC4 proteins of the invention encompass APOBEC4 proteins modified at the C-terminus by addition of at least one amino acid sequence selected from the group consisting of the V5 tag, the c-myc tag and the HA tag or an amino acid sequence homologous thereto. Furthermore, amino acid sequences that are homologous to the V5 tag, the c-myc tag or, preferably, the HA tag, preferably, at least 90% identical thereto, are also explicitly mentioned as amino acid sequences that may be used to modify the APOBEC4 protein according to the present invention. Further disclosed are APOBEC4 proteins that are mutated within the zinc-finger domain, preferably, by E95A, C127A and C134A amino acid substitutions. The most preferred modified APOBEC4 proteins of the present invention are HA-APOBEC4, APOBEC4-HA, APOBEC-3xHA. Also disclosed is APOBEC4 complete lacking the poly-lysine tail.

Nucleic acids coding for the APOBEC4 proteins of the present invention, for example plasmids constructed for the expression such proteins, are also encompassed. Furthermore, all proteins and nucleic acids homologous to the APOBEC4 protein or nucleic acids of the present invention are also encompassed. Preferably, the amino acid sequence of the homologous protein or the nucleic acid sequence of the homologous nucleic acid is at least 90%, 95%, 97%, 99%, or 99.5% identical to the APOBEC4 proteins or the nucleic acids of the present invention.

Given the working hypothesis outlined above, a multitude of additional modified APOBEC4 proteins and functional homologs thereof are obviously derivable for the skilled person. Also disclosed is KRR1 (GeneBank access number NP_008974), the human HIV-1 rev binding protein 2 is given that likewise comprises a poly-lysine tail. Thus, KRR1-HA, HA-KRR1, KRR1-3xHA and KRR1 with a truncated or mutated poly-lysine tail are also disclosed. Furthermore, it is disclosed that the poly-lysine tail of APOBEC4 itself, either in unmodified form or comprising amino acid substitutions may compete for at least one of the factors required to form a functional APOBEC4 complex recruited during viral reproduction.

Cells

While the present invention has exemplarily been described and studied in 293T cells and HeLa cells, it is contemplated that other cells may be used according to the teachings of the present invention. Preferably, the cells used in accordance with the present invention are human cells, macrophages, T cells, stem cells, autologous cells, as well as progenitor cells or cell lines thereof. Most preferably, the cells are 293T cells or HeLa cells.

Medical Uses and Pharmaceutical Compositions

The constructs of the present invention can directly be used in a variety of medical applications or for the manufacture or medicaments.

In one aspect of the invention the N-terminally stabilized/modified APOBEC4 proteins of the invention can be expressed in a cell producing a retrovirus or viral particles thereof and be used to augment production of retroviruses or viral particles by said cell. Therefore, the present invention provides a method for increasing the production of retroviruses, *inter alia,* HIV, SIV, FIV, EIAV, MLV, HIV-1, HIV-2, SIVagm, SIVmac, spuma virus or viral particles thereof in a cell that can subsequently be isolated and used for purposes of vaccination against such retroviruses.

In another embodiment, the present invention can be used for gene therapy purposes, *inter alia,* to treat, inhibit or decrease production of a retrovirus in a mammal. In one embodiment, the viruses or viral particles of the present invention, or pharmaceutical preparations thereof, can be administered to a mammal, preferably, a human or a horse. If the viruses or viral particles exhibit the same tropism as the retrovirus that is to be treated then selective infection of the cells of the mammal which are the target cells of the retrovirus to be treated by the viruses or viral particles of the present invention will result. Upon cell entry the viruses or viral particles of the invention express the C-terminally modified APOBEC4 protein which subsequently leads to a decrease in viral production from said cell. As an example, if HIV-1 is to be treated viruses derived from HIV-1 and comprising the genetic information for a C-terminally modified APOBEC4 protein, preferably APOBEC4-3xHA, are administered to the mammal. As the administered viruses are derived from HIV-1 they exhibit the same tropism as an HIV-1 virus and will consequently infect the same cell-types, for example T cells. The infected cells will now express the C-terminally modified APOBEC4 protein, which will decrease production of HIV-1. It is evident to the skilled person that infections with any other retrovirus, including HIV, SIV, FIV, EIAV, MLV, HIV-2, spuma virus, SIVagm and SIVmac, can be treated in this manner.

It is disclosed that the viruses or viral particles of the present invention harbouring the genetic information for C-terminally modified APOBEC4 protein according to the present invention may be used to transduce retroviral target cells *ex vivo* and then reintroduce these into the mammal to be treated. In case of HIV the target cells are preferably T helper cells and/or macrophages or their progenitor cells.

It is disclosed that the transfer construct may be transduced into the target cells of the retrovirus by means of the viruses or viral particles of the present invention encodes sh/siRNA which targets the endogenous APOBEC4 and further encodes a codon optimized therapeutic APOBEC4, for example APOBEC-3xHA or APOBEC-HA, which is not affected by the sh/siRNA molecules. The transfer construct encodes an antibody which targets the endogenous APOBEC4 and may or may not encode a modified therapeutic APOBEC4 protein, which is not affected by the antibodies

Pharmaceutical compositions based on the constructs of the present invention can be formulated in any conventional manner using one or more physiologically acceptable carriers or excipients. Thus, the viruses or viral particles, nucleic acids, proteins and compounds inhibiting the transcription and/or expression of APOBEC4 or the proper functioning of APOBEC4 of the present invention may be formulated for administration by, for example, injection, inhalation or insulation (either through the mouth or the nose) or by oral, buccal, parenteral or rectal administration.

The pharmaceutical compositions of the present invention can be formulated for a variety of modes of administration, including systemic, topical or localized administration. Techniques and formulations can be found in, for example, Remrnington's Pharmaceutical Sciences, Meade Publishing Co., Easton, Pa. For systemic administration, injection is preferred, including intramuscular, intravenous, intraperitoneal, and subcutaneous. For the purposes of injection, the pharmaceutical compositions of the present invention can be formulated in liquid solutions, preferably in physiologically compatible buffers, such as Hank's solution or Ringer's solution. In addition, the pharmaceutical compositions may be formulated in solid form and redissolved or suspended immediately prior to use. Lyophilized forms of the pharmaceutical composition are also suitable.

For oral administration, the pharmaceutical compositions of the present invention may take the form of, for example, tablets or capsules prepared by conventional means with pharmaceutically acceptable excipients such as binding agents (e.g. pregelatinized maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycolate); or wetting agents (e.g. sodium lauryl sulfate). The tablets can also be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, cellulose derivatives or hydrogenated edible fats); emulsifying agents (e.g. lecithin or acacia); non-aqueous vehicles (e.g. ationd oil, oily esters, ethyl alcohol or fractionated vegetable oils); and preservatives (e.g. methyl or propyl-p-hydroxybenzoates or sorbic acid). The preparations can also contain buffer salts, flavoring, coloring and sweetening agents as appropriate.

The pharmaceutical compositions can be formulated for parenteral administration by injection, e.g. by bolus injection or continuous infusion. Formulations for injection can be presented in a unit dosage form, e.g. in ampoules or in multi-dose containers, with an optionally added preservative. The pharmaceutical compositions can further be formulated as suspensions, solutions or emulsions in oily or aqueous vehicles, and may contain other agents including suspending, stabilizing and/or dispersing agents.

Additionally, the pharmaceutical compositions can also be formulated as a depot preparation. These long acting formulations can be administered by implantation (e.g. subcutaneously or intramuscularly) or by intramuscular injection. Thus, for example, the compounds may be formulated with suitable polymeric or hydrophobic materials (e.g. as an emulsion in an acceptable oil) or ion exchange resins, or as sparingly soluble derivatives, for example, as a sparingly soluble salt. Other suitable delivery systems include microspheres, which offer the possibility of local noninvasive delivery of drugs over an extended period of time. This technology can include microspheres having a precapillary size, which can be injected via a coronary catheter into any selected part of an organ without causing inflammation or ischemia. The administered therapeutic is then slowly released from the microspheres and absorbed by the surrounding cells present in the selected tissue.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, bile salts, and fusidic acid derivatives. In addition, detergents may be used to facilitate permeation. Transmucosal administration can occur using nasal sprays or suppositories.

Screenin Assays for Antiviral Compounds

The key role, APOBEC4 plays in retroviral reproduction makes it a prime target for antiretroviral drugs. Thus, APOBEC4 can be used in an assay to screen for compounds that inhibit APOBEC4 transcription and/or expression, and/or the function of APOBEC4 in retroviral reproduction.

As it was found that endogenous APOBEC4 is expressed in, *inter alia,* 293T cells or HeLa cells. Such cells could be used in a high throughput assay to screen for compounds that inhibit APOBEC4 transcription and/or expression. It is apparent to the skilled person that also cells or cell lines expressing exogenous APOBEC4 could be used in such an assay.

In one embodiment of the invention, the method for screening for antiviral compounds comprises the steps of contacting a cell expressing a human APOBEC4 protein with a test compound and determining whether the test compound affects APOBEC4 transcription and/or expression of said cell, wherein a decrease in APOBEC4 transcription and/or expression indicates that the test compound is an antiviral compound. In a preferred embodiment the APOBEC4 transcription and/or expression of a first cell in the presence of the test compound is compared to the APOBEC4 transcription and/or expression of a second cell not contacted with the test compound, wherein a decrease in APOBEC4 transcription and/or expression of the first cell indicates that the test compound is a potential antiviral compound. Preferably, the cells used in the screening method are human cells, macrophages, T cells, stem cells, autologous cells, as well as progenitor cells or cell lines thereof. Most preferably, the cells are 293T cells or HeLa cells. Preferably, the APOBEC4 transcription and/or expression is determined by RT-PCR, Northern blotting and western blotting, respectively.

In a further embodiment the invention is directed to a method of screening for screening for antiviral compounds. Candidate compounds may initially be selected or chemically designed based on their predicted or determined binding to the APOBEC4 protein. The predicted binding of a compound, e.g. a protein, to APOBEC4 can be computed using docking algorithms known to the skilled person which produce many possible structures of a protein-protein or protein-compound complex and in most cases some of them resemble the correct structure within an r.m.s.d. of <3 A. The BIOCOR binding analysis can be used to measure the binding of a given compound to APOBEC4. 293T cells are then infected with a retrovirus and the titer of the retrovirus is determined in the presence and the absence of one or more of the selected candidate compounds. A decrease in the titer indicates that the candidate compound interferes with the assembly of the retrovirus by interaction with the APOBEC4 protein and thus is a potential antiretroviral drug.

As a further example, siRNA or shRNA molecules directed against APOBEC4 mRNA are used as antiretroviral test compounds. It is expected that knock-down of APOBEC4 by such siRNA or shRNA molecules will greatly reduce the release of viruses by the infected cell. Therefore, siRNA or shRNA molecules may be used as antiretroviral drugs.

### Examples

### Generation of Plasmids

Plasmids for the expression of APOBEC4 and modified APOBEC4 were generated by PCR, starting from the APOBEC4 cDNA (GeneBank accession no NM_203454) from human germ cells. The unmodified APOBEC4 protein and the modified APOBEC4 fusion protein comprising an N-terminal addition of a tag were expressed from the pcDNA3.1/Zeo(+) plasmid (Invitrogen). The modified APOBEC4 fusion protein comprising a C-terminal addition of a tag was expressed from the pMHC3xHA plasmid.

To exemplify, the following primers were used for the generation of phA4-3xHA (SEQ ID NOs: 1 and 2), an expression plasmid based on the pMHC3xHA plasmid and expressing the human APOBEC4 protein modified with three HA tags fused to the C-terminus: 5' primer: cag gcg gta cca gcc tgg aga caa att gat gg (SEQ ID NO: 7), 3' primer: gac ttg gat ccc ctt tct tcc ctt tct tct tct tct ttt cat ctg cct cct tgc tac (SEQ ID NO: 8).

For the generation of the pHA-hA4_cDNA3.1Zeo(+) expression plasmid (SEQ ID NOs: 3 and 4), based on the pcDNA3.1/Zeo(+) plasmid and expressing the human APOBEC4 protein modified with a HA tag fused to the N-terminus, the following primers were used: 5' primer: cgg atc cct agc aat ggg ata tcc ata cga tgt tcc aga tta cgc tga gcc cat ata tga gga gta cc (SEQ ID NO: 9), 3' primer: gaa ttc ttt att tct tcc ctt tct tct tct tc (SEQ ID NO: 10).

For the generation of the phA4_cDNA3.1Zeo(+) expression plasmid (SEQ ID NOs: 5 and 6), likewise based on the pcDNA3.1/Zeo(+) plasmid and expressing the human APOBEC4 protein, the following primers were used: 5' primer cgg atc cct agc aat gga gcc cat ata tg (SEQ ID NO: 11), 3' primer gaa ttc ttt att tct tcc ctt tct tct tct tc (SEQ ID NO: 12).

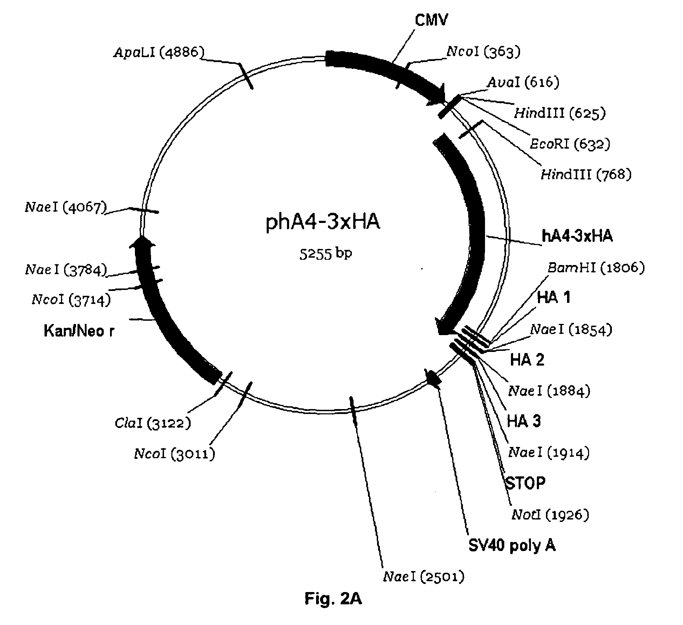

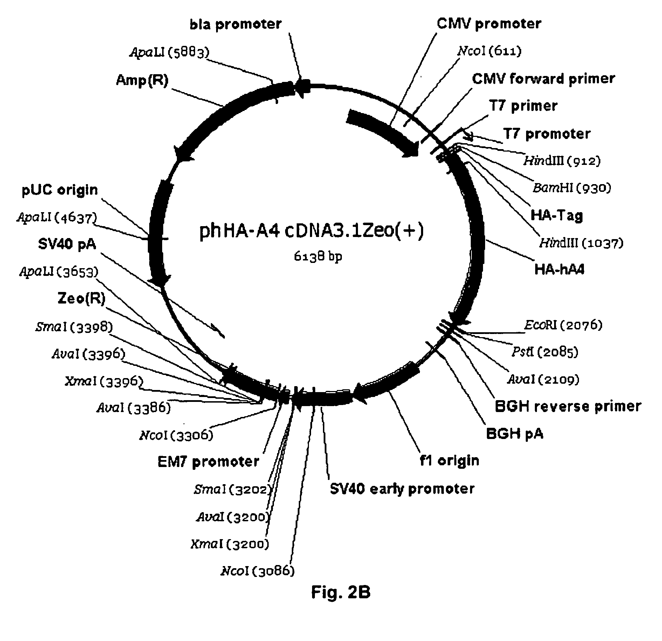

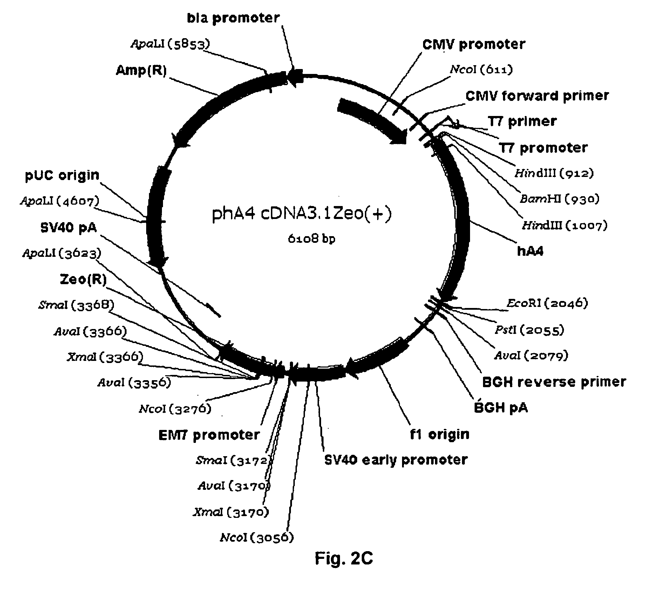

The amplicons generated were cloned into the respective plasmid vectors using the *Bam*HI and *EcoR*I restriction enzymes. **Figures 2A-C** show plasmid maps of the three exemplified expression vectors phA4-3xHA, pHA-hA4_cDNA3.1Zeo(+), and phA4_cDNA3.1Zeo(+), respectively.

The skilled person will know, how to generate different expression vectors comprising, *inter alia,* different tags, for example the V5 or c-myc tag, a different number of tags, or how to introduce mutations into the APOBEC4 gene to, for example, truncate or completely delete the C-terminal poly-lysine tail, or insert mutations to zinc-finger domain.

### Cell Culture

In the following cell culture is exemplified for 293T cells. The use of different cells is envisaged and encompassed by the teaching of the present invention.

Adherend 293T cells were cultured in DMEM (GIBCO/BRL). For cell passage, cells were detached using a 1 mM EDTA/PBS solution and transferred into fresh medium (1:10) twice a week. Cells were washed using PBS (137 mM NaCl, 2.7 mM KCL, 4.3 mM Na₂HPO₄, 1.4 mM KH₂PO₄). All culturing media were supplemented with 10% fetal calf serum (FCS, Biochrom KG), 2mM L-glutamine (Biochrom KG) and antibiotics (100 U/ml penicillin, 50 mg/ml streptomycine [Biochrome KG]). FCS was incubated for 30 min at 46° C for inactivation of components of the complement system. Culturing of the cells was carried out in a cell incubator (BBD 6220, Heraeus) at 37° C and 5% CO₂.

### Transfection and Production of Viral Cell Culture Supernatant

One day prior to transfection, 0.8 x 10⁶ 293T cells were seeded per well of cell culture plate. For transfection, LipofectamineLTX (Invitrogen) was used in a 2:1 ratio (µl LipofectamineLTX : µg plasmid DNA) according to the manufacturer's recommendations.

For the production of viral cell culture supernatant, cells were co-transfected with 1 µg plasmid DNA of a proviral genome, if required 0,5 µg expression vector pMD.G (expressing the VSV-G protein), and 0-3 µg of the respective APOBEC4 plasmid. If required, pCDNA3.1 plasmid was added to keep the total amount of DNA added at 4 µg. Harvesting of viral supernatant was carried out 48 hrs after transfection. Cell debris contained in the supernatants was removed by filtration using filters with a pore size of 45 µm (Sartorius). The filtrate was stored at -80 °C.

### Luciferase Reporter Virus Test

Reporter viruses allow a fast and quantitative analysis of interactions of the cellular and viral components as well as of antiviral factors. To study the influence of APOBEC4 on the virus-host interaction the VSV-G pseudotyped reporter virus NL-luc R-E- was employed. NL-luc R-E- is derived from the HIV-1 variant NL4-3 and features a deletion of the *env* and *net* genes. Furthermore, NL-luc R-E- carries the *luciferase* reporter gene of *Photinus pyralis.*

293T cells were transfected with equal amount of the different plasmids. 48 hrs after transfection and production of viruses/viral particles with or without the (modified) APOBEC4 of the present invention, viruses/viral particles were harvested and cell lysates (of about 2 x 10⁴ cells) were analyzed for luciferase expression using the Steadylite HTS Kit (Perkin Elmer) and according to the manufacturer's recommendations.

### Denaturing Polyacrylamide Gelelectrophoresis (SDS-PAGE) and Western Blot

For cell lysis, 293T cells were washed in 1 ml PBS after harvesting of the viruses/viral particles, detached using PBS/EDTA, pelletted (250 rpm, 5 min, RT) and lysed during an incubation of 5 min on ice after addition of RIPA lysis buffer (25 mM TRIS, pH 8, 137 mM NaCl, 1% glycerine, 0.1 % SDS, 0.5% Na-deoxycholate, 1 % NaPO₄). After a centrifugation step of 10 min at 14000 rpm and 4° C, protein concentration of the supernatant was carried out according to Bradford: 5 µl of the supernatant were added to 1000 µl of Bradford reagent (Bio-Rad, Hercules, USA) and the absorption at 595 nm was measured using a spectrometer (Gene Quant II, RNA/DNA-Calculator, Pharmacia Biotech).

For SDS-PAGE and western blotting, cell lysate corresponding to 30 µg of total protein was loaded to NuPAGE gradient gels (4-12%; Invitrogen) and electrophoresis was carried out according to the manufacturer'S recommendations. PVDF membrane (Invitrogene) was incubated with blocking solution (PBS, 5% milk powder, 0.5% Tween20 [Roth]) for 1 hr or over night at 4° C to prevent an unspecific binding of the primary antibody. As primary antibodies, mouse anti-HA (1:5000 in blocking solution, MMS-101 P, Covance), mouse anti-αTubulin (1:10000, B5-1-2, Sigma) or mouse anti-p24 (1:800, 183-H12-5C, The following reagent was obtained through the NIH AIDS Research and Reference Reagent Program, Division of AIDS, NIAID, NIH: HIV-1 p24 Monoclonal Antibody (183-H12-5C) from Dr. Bruce Chesebro and Kathy Wehrly). As secondary antibody, an antimouse horseradish peroxidase conjugated antibody (1:7500 GE Healthcare) was used. Before and after each incubation period, the membrane was first washed with washing solution 1 (PBS, 0.5 % TWeen20) twice and subsequently incubated in washing solution 2 (PBS, 0.1 % Tween20) for 25 min. Lumigen PS-3 Acridan (ECL plus Western Blotting Detection System, GE Healthcare) was used as the substrate, whose enzymatic conversion leads to light emission. Detection of the light emission was carried out using Amersham Hyperfilm ECL (GE Healthcare) in developing solution (Curix 60, Agfa).

### Example 1: Modulation of HIV-1 NL4-3 Virus Production by APOBEC4 Expression

In the following, 293T cells are used as an exemplary cell type and HIV-1 is used as an exemplary virus.

293T cells were co-transfected as described above with an expression vector comprising the proviral nucleic acid sequence of the HIV-1 variant HIV-1 NL4-3 and expression vectors coding for the unmodified human APOBEC4 protein, as well as modified human APOBEC4 proteins HA-APOBEC4, APOBEC-HA and APOBEC4-3xHA, respectively. Subsequently, production of viruses in the viral supernatant (V) and cell lysates (C), respectively was determined in a reverse transcriptase assay (RT-PCR; not shown) and western blotting. Specifically, expression of the HIV antigen p24 and the HA tag and tubulin was assessed.

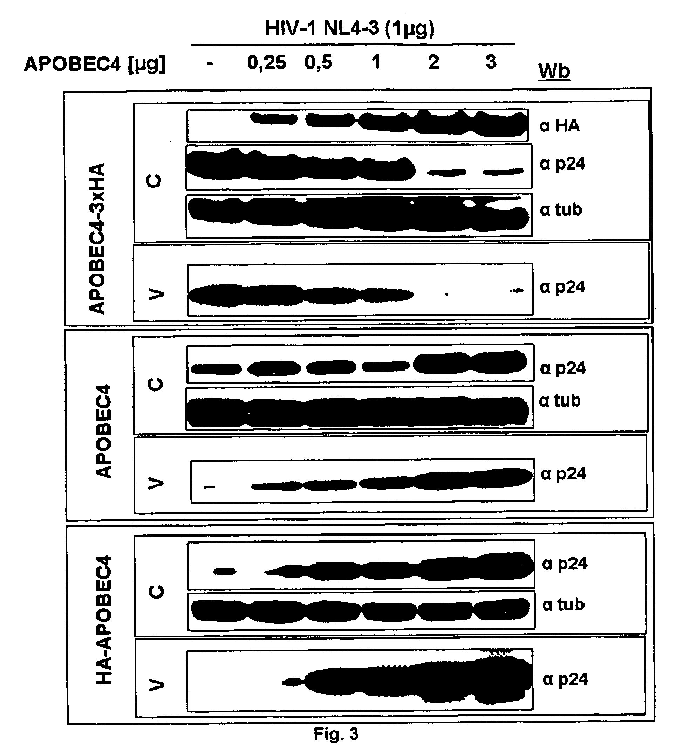

Figure 3 shows the results of the western blotting. Co-transfection of the cells with HIV-1 NL4-3 and the expression plasmid coding for the unmodified APOBEC4 protein lead to an increase in viral production in both viral supernatant (V) and cell lysates (C) that directly and linearly correlated to the amount of APOBEC4 plasmid used during co-transfection (Fig. 3, middle panel).

Co-transfection with the expression plasmid coding for HA-APOBEC4 protein lead to an augmented increase in viral production in both viral supernatant and cell lysates compared to co-transfection with unmodified APOBEC4 protein. Viral production that resulted in co-transfection with HA-APOBEC4 expression plasmid was increased 5-10-fold compared to that of co-transfection with APOBEC4 expression plasmid. Likewise, the amount of produced viruses directly and linearly correlated to the amount of HA-APOBEC4 plasmid used during co-transfection (Fig. 3, bottom panel).

Co-transfection with the expression plasmid coding for APOBEC4-3xHA protein lead to a drastic decrease (up to 95 %) in viral production in both viral supernatant and cell lysates compared to co-transfection with unmodified APOBEC4 protein. Likewise, the degree of inhibition of virus production directly and linearly correlated to the amount of APOBEC4-3HA plasmid used during co-transfection (Fig. 3, top panel). Similar results were obtained using an expression plasmid coding for APOBEC4-HA (not shown).

Together the results indicate that an APOBEC4 complex is recruited by the virus-infected cell during virus production. If additional APOBEC4 protein is present, virus production is increased. Expression of a modified APOBEC4 protein that is stabilized by modification of the N-terminus, *inter alia,* by addition of an amino acid sequence, for example, the HA tag, leads to an increased amount of APOBEC4 complex in the cell and thus an increased production of the virus by said cell. Expression of a modified APOBEC4 protein whose binding to the C-terminal poly-lysine tail is decreased or inhibited, *inter alia,* by modification of the C-terminus by, for example addition of an amino acid sequence as the HA tag or three HA tags, leads to a decreased amount of APOBEC4 complex in the cell and thus a decreased production of viruses by said cell.

Furthermore, and as evident from the results of, e.g., the western blotting, the pro- and anti-viral effects, respectively according to the present invention directly correlate with the amount of APOBEC4 expression vector used for transfection. In other words, the ration between expression vector coding for the proviral genome and plasmid vector encoding the (modified) APOBEC4 protein determines the level of increase or decrease of viruses by the transfected cell. As the amount of DNA that can be transfected into a cell is limited due to the cytotoxicity of the transfection reagent, cell lines stably expressing the (modified) APOBEC4 protein were generated and subsequently transfected with expression vectors encoding various proviral genomes. In such cell lines the pro- and anti-viral effects of the present invention were clearly enhanced.

### Example 2: Modulation of HIV-1 NL-luc R-E- Virus Production by APOBEC4 Expression

The experiments of Example 1 were repeated using the HIV-1 NL4-3 derivate NL-luc R-E-, pseudotyped with the VSV-G protein.

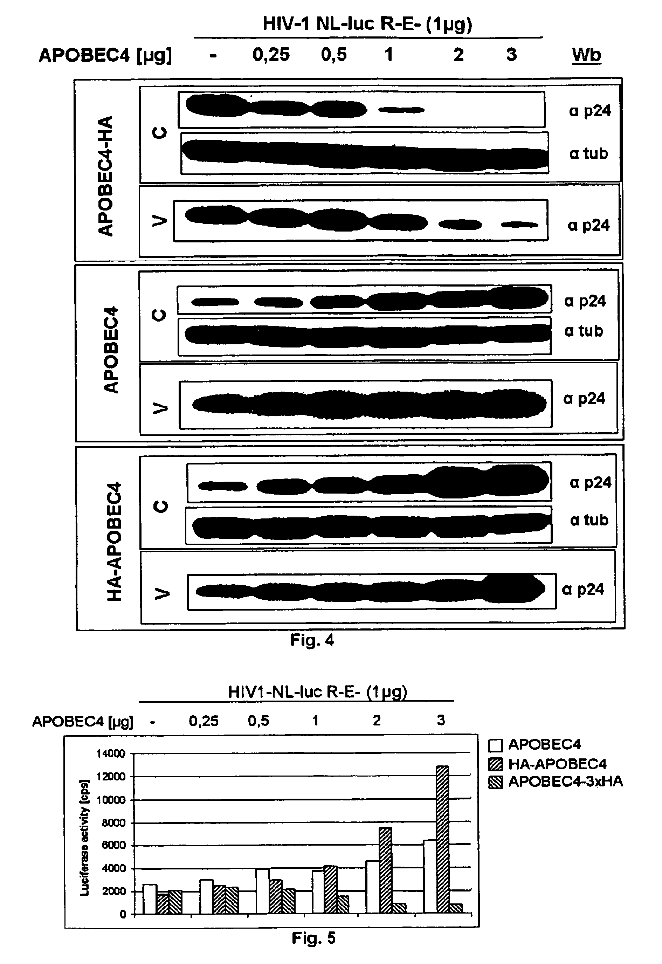

Figure 4 shows the results of the western blotting that confirm the findings from Example 1. Co-transfection of the cells with an expression plasmid encoding the reporter virus and the expression plasmid coding for the unmodified APOBEC4 protein lead to an increase in viral production in both viral supernatant and cell (Fig. 4, middle panel); co-transfection with the expression plasmid coding for HA-APOBEC4 protein lead to an augmented increase in viral production (Fig. 4, bottom panel) and co-transfection with the expression plasmid coding for APOBEC4-3xHA protein lead to a drastic decrease in viral production (Fig. 4, top panel). Likewise, the degree of increase or decrease in virus production directly and linearly correlated to the amount of APOBEC4 expression plasmid used during co-transfection. Similar results were obtained using an expression plasmid coding for APOBEC4-HA (not shown).

Together the results indicate that the pro- and antiviral effects disclosed by the present invention are not restricted to wild type proviral genomes but are also applicable to, for example, pseudotyped viruses.

### Examples 3: Reporter Virus Assays

The VSV-G pseudotyped reporter virus NL-luc R-E- was employed to verify the results determined by western blotting. 293T cells were transfected as described above, viruses were harvested and cell lysates of about 2 x 10⁴ cells were analyzed for luciferase expression.

Figure 5 shows the results of the reporter virus assays and demonstrates that the expression of the *luciferase* reporter gene (instead of the viral *nef* gene) depends on APOBEC4 expression in the same manner as the production of viruses depends on APOBEC4 expression. Co-transfection of the cells with NL-luc R-E- and the expression plasmid coding for the unmodified APOBEC4 protein lead to an increase in luciferase activity that directly and linearly correlated to the amount of APOBEC4 plasmid used during co-transfection.

Co-transfection with the expression plasmid coding for HA-APOBEC4 protein lead to an augmented increase in luciferase activity compared to co-transfection with unmodified APOBEC4 protein. Likewise, the increase in luciferase activity directly and linearly correlated to the amount of HA-APOBEC4 plasmid used during co-transfection.

Co-transfection with the expression plasmid coding for APOBEC4-3xHA protein lead to a drastic decrease in luciferase activity compared to co-transfection with unmodified APOBEC4 protein. Again, the degree of decrease in luciferase activity directly and linearly correlated to the amount of APOBEC4-3HA plasmid used during co-transfection. Similar results were obtained using an expression plasmid coding for APOBEC4-HA (not shown).

Together these results demonstrate that the reporter virus assays of the present invention allow for a fast and quantitative analysis of interactions of the cellular and viral components as well as of antiviral factors. Furthermore, the results demonstrate that the pro- and anti-viral effects of the present invention are also applicable for viruses that are genetically modified, for example, to carry a heterologous gene.

### Example 4: Modulation of Production of Different Virus Strains by APOBEC4 Expression

The experiments of the foregoing Examples were repeated using a truncated virus genome coding only for the structural proteins and enzymes (Gag, Pol). Furthermore, SIVagm, SIVmac and MLV were tested as additional different virus strain.

For all tested viruses the results of the foregoing Examples could be verified. In each case, co-transfection of the cells with an expression plasmid encoding the proviral genome and the expression plasmid coding for the unmodified APOBEC4 protein lead to an increase in viral production in both viral supernatant and cell; co-transfection with the expression plasmid coding for HA-APOBEC4 protein lead to an augmented increase in viral production and co-transfection with the expression plasmid coding for APOBEC4-3xHA or APOBEC3-HA protein lead to a drastic decrease in viral production. In each case, the degree of increase or decrease in virus production directly and linearly correlated to the amount of APOBEC4 expression plasmid used during co-transfection.

Together the results indicate that the pro- and antiviral effects disclosed by the present invention are not restricted to the HIV-1 virus but are also applicable to, for example, truncated HIV viruses and/or various other retroviruses or lentiviruses. Therefore, the present invention provides means to increase or decrease, i.e. modulate the production of retroviruses and lentiviruses or particles thereof.

### Example 5: APOBEC4 as a Target for Antiretroviral Drugs

293T cells or HeLa cells are contacted with a candidate compound and the amount of APOBEC4 transcript and/or APOBEC4 protein is determined by RT-PCR, Northern blotting and western blotting, respectively. A decrease of the APOBEC4 transcript and/or APOBEC4 protein in the presence of the candidate compound as compared to the absence of the candidate compound indicates that the candidate compound is a potential inhibitor of APOBEC4 transcription and/or expression and thus a potential antiretroviral drug.

In another assay, 293T or HeLa cells infected with a retrovirus are contacted with one or more test compound, for example siRNA or shRNA molecules directed against APOBEC4 mRNA, and the titer of the retrovirus is determined in the presence and the absence of a test compound. A decrease in the titer indicates that the test compound interferes with the assembly of the retrovirus by interaction with the APOBEC4 protein and thus is a potential antiretroviral drug.

### References

- ROGOZIN, IB, et al. APOBEC4, a new member of the AID/APOBEC family of polynucleotide (deoxy)cytidine deaminases predicted by computational analysis. Cell Cycle. 2005, vol.4, no.9, p.1281-5.
- CONTICELLO, SG, et al. Evolution of theAID/APOBEC family of polynucleotide (deoxy)cytidine deaminases. Mol Biol Evol. 2005, vol.22, no.367, p.77.
- HOLMES, RK, et al. APOBEC-mediated viral restriction: not simply editing? Trends Biochem Sci. 2005, vol.32, no.3. p.118-128
- WO 2005/117947
- WO 2006/065377
- KIM, VN, et al. Minimal requirement for a lentivirus vector based on human immunodeficiency virus type 1. J Virol.. 1998, vol.72, no.1, p.811-6.
- ALTSCHUL, SF, et al. Basic local alignment search tool. J Mol Biol. 1990, vol.215, no.3, p.403-10.
- PERASON, WR, et al. Improved tools for biological sequence comparison. Proc Natl Acad Sci U S A. 1988, vol.85, no.8, p.2444-8.
- VIGNA, E, et al. Lentiviral vectors: excellent tools for experimental gene transfer and promising candidates for gene therapy. J Gene Med. 2000, vol.2, no.5, p.308-16.
- PALU, G, et al. Progress with retroviral gene vectors. Rev Med Virol. 2000, vol.10, no.3, p.185-202.

## Claims

1. A modified human APOBEC4 protein **characterized in that** the N-terminus is modified to stabilize the APOBEC4 protein or the C-terminus is modified, wherein the modification of the N-terminus comprises addition of at least one amino acid sequence selected from the group consisting of the V5 tag, the c-myc tag and the HA tag or an amino acid sequence homologous thereto, and wherein the modification of the C-terminus comprises addition of at least one amino acid sequence selected from the group consisting of the V5 tag, the c-myc tag and the HA tag or an amino acid sequence homologous thereto.

2. The modified APOBEC4 protein according to claim 1, wherein the at least one amino acid sequence added to the N-terminus is HA tag or a sequence at least 90% identical thereto.

3. The modified APOBEC4 protein according to claim 1, wherein the at least one amino acid sequence added to the C-terminus is HA tag or 3 HA tags or a sequence at least 90% identical thereto.

4. A cell expressing the modified APOBEC4 protein according to claims 1-3.

5. The cell according to claim 4, wherein the cell further produces a retrovirus or viral particles thereof.

6. The cell according to claim 5, wherein the retrovirus is a lentivirus and/or is a replication deficient virus.

7. The cell according to claim 6, wherein the N-terminus of the APOBEC4 protein is modified to stabilize the APOBEC4 protein.

8. The cell according to claims 4-7, wherein the cell is selected from the group consisting of a 293T cell, a HeLa cell, a T-helper cell, and a macrophage.

9. A nucleic acid coding for the modified APOBEC4 protein according to anyone of claims 1-3 or the complement thereof.

10. A nucleic acid comprising the nucleic acid sequence according to claim 9.

11. A nucleic acid or vector for somatic gene therapy comprising the nucleic acid according to claim 10, wherein the nucleic acid coding for APOBEC4 is operably linked to a promoter.

12. A vaccine comprising the cell according to claim 7 and an acceptable pharmaceutical carrier.

13. A retroviral replication deficient particle comprising the nucleic acid according to claims 10-11, wherein the nucleic acid is an RNA.

14. The retroviral particle according to claim 13, wherein the retroviral particle is derived from HIV, HIV-1, HIV-2, SIV, SIVagm, SIVmac, spuma virus, FIV, EIAV or MLV.

15. An *in vitro* method for decreasing the production of retroviruses or lentiviruses or viral particles thereof of a cell producing a retrovirus, lentivirus or vector particles thereof, the method comprising expressing a modified APOBEC4 protein according to claims 1 and 3 in said cell, wherein the C-terminus of the APOBEC4 protein is modified.

16. The method according to claim 15, wherein the production of retroviruses or viral particles thereof is decreased by at least 20%.

17. A method for producing retroviral particles, the method comprising cultivating a cell according to claim 7.

18. The use of a cell according to claim 7, the nucleic acid according to claim 11 or the retroviral particle according to claims 13-14 for the manufacturing of a medicament.

19. The use of a cell according to claim 7, the nucleic acid according to claim 11 or the retroviral particle according to claims 13-14 for the manufacturing of a medicament for the treatment of or vaccination against an infection by a retrovirus.

20. A method for screening for antiviral compounds, the method comprising the steps of:
contacting a cell expressing a human APOBEC4 protein with a test compound; and
determining whether the test compound affects APOBEC4 transcription and/or expression of said cell,
wherein a decrease in APOBEC4 transcription and/or expression of said cell indicates that the test compound is an antiviral compound.

21. The method according to claim 20, wherein the APOBEC4 transcription and/or expression of a first cell in the presence of a test compound is compared to the APOBEC4 transcription and/or expression of a second cell in the absence of said test compound,
wherein a decrease in APOBEC4 transcription and/or expression of the first cell compared to the second cell indicates that the test compound is an antiviral compound.

22. The method according to claims 20 or 21, wherein the APOBEC4 transcription and/or expression is determined by RT-PCR, Northern blotting and/or western blotting.

23. A method for screening for antiviral compounds, the method comprising the steps of
contacting a cell expressing a retrovirus and an APOBEC4 protein with a test compound; and
determining the titer of the retrovirus in the presence and the absence of the test compound,
wherein a decrease in the titer indicates that the test compound is an antiviral compound.

24. The method according to anyone of claims 20-23, wherein the test compound is an siRNA or shRNA molecule directed against APOBEC4 mRNA.

## Patentansprüche

1. Ein modifiziertes humanes APOBEC4-Protein charakterisiert **dadurch**, dass der N-Terminus modifiziert ist, um das APOBEC4-Protein zu stabilisieren, oder der C-Terminus modifiziert ist, wobei die Modifizierung des N-Terminus das Hinzufügen von mindestens einer Aminosäuresequenz ausgewählt aus der Gruppe bestehend aus dem V5-Tag, dem c-myc-Tag und dem HA-Tag oder einer dazu homologen Aminosäuresequenz umfasst und wobei die Modifizierung des C-Terminus das Hinzufügen von mindestens einer Aminosäuresequenz ausgewählt aus der Gruppe bestehend aus dem V5-Tag, dem c-myc-Tag und dem HA-Tag oder einer dazu homologen Aminosäuresequenz umfasst.

2. Das modifizierte APOBEC4-Protein gemäß Anspruch 1, wobei die mindestens eine an den N-Terminus angefügte Aminosäuresequenz ein HA-Tag oder eine mindestens zu 90% dazu identische Sequenz ist.

3. Das modifizierte APOBEC4-Protein gemäß Anspruch 1, wobei die mindestens eine an den C-Terminus angefügte Aminosäuresequenz ein HA-Tag oder 3 HA-Tags oder eine mindestens zu 90% dazu identische Sequenz ist.

4. Eine Zelle, die das modifizierte APOBEC4-Protein gemäß Ansprüchen 1-3 exprimiert.

5. Die Zelle gemäß Anspruch 4, wobei die Zelle weiter einen Retrovirus oder virale Partikel davon produziert.

6. Die Zelle gemäß Anspruch 5, wobei der Retrovirus ein Lentivirus und/oder ein replikationsdefizienter Virus ist.

7. Die Zelle gemäß Anspruch 6, wobei der N-Terminus des APOBEC4-Proteins modifiziert ist, um das APOBEC4-Protein zu stabilisieren.

8. Die Zelle gemäß Ansprüchen 4-7, wobei die Zelle ausgewählt ist aus der Gruppe bestehend aus einer 293T-Zelle, einer HeLa-Zelle, einer T-Helferzelle und einem Makrophagen.

9. Eine Nukleinsäure, die für das modifizierte APOBEC4-Protein gemäß einem der Ansprüche 1-3 oder sein Komplement kodiert.

10. Eine Nukleinsäure umfassend die Nukleinsäuresequenz gemäß Anspruch 9.

11. Eine Nukleinsäure oder ein Vektor für somatische Gentherapie umfassend die Nukleinsäure gemäß Anspruch 10, wobei die für APOBEC4 kodierende Nukleinsäure operabel an einen Promotor gekoppelt ist.

12. Ein Impfstoff umfassend die Zelle gemäß Anspruch 7 und einen akzeptablen pharmazeutischen Träger.

13. Ein retrovirales replikationsdefizientes Partikel umfassend die Nukleinsäure gemäß Ansprüchen 10-11, wobei die Nukleinsäure eine RNS ist.

14. Das retrovirale Partikel gemäß Anspruch 13, wobei das retrovirale Partikel von HIV, HIV-1, HIV-2, SIV, SIVagm, SIVmac, Spumavirus, FIV, EIAV oder MLV abgeleitet ist.

15. Ein *in-vitro*-Verfahren für die Verringerung der Produktion von Retroviren oder Lentiviren oder viralen Partikeln davon in einer Zelle, die Retrovirus, Lentivirus oder Vektorpartikel davon produziert, das Verfahren umfassend Expression des modifizierten APOBEC4-Proteins gemäß Ansprüchen 1 und 3 in der Zelle, wobei der C-Terminus des APOBEC4-Proteins modifiziert ist.

16. Das Verfahren gemäß Anspruch 15, wobei die Produktion von Retroviren oder viralen Partikeln davon um mindestens 20% verringert wird.

17. Ein Verfahren zur Produktion retroviraler Partikel, die Methode umfassend Kultivieren einer Zelle gemäß Anspruch 7.

18. Die Verwendung einer Zelle gemäß Anspruch 7, der Nukleinsäure gemäß Anspruch 11 oder des retroviralen Partikels gemäß Ansprüchen 13-14 zur Herstellung eines Medikamentes.

19. Die Verwendung einer Zelle gemäß Anspruch 7, der Nukleinsäure gemäß Anspruch 11 oder des retroviralen Partikels gemäß Ansprüchen 13-14 zur Herstellung eines Medikamentes zur Behandlung von oder Impfung gegen eine Infektion mit einem Retrovirus.

20. Ein Verfahren zur Ausleseprüfung von antiviralen Verbindungen, das Verfahren umfassend die Schritte: Kontaktieren einer Zelle, die das humane APOBEC4-Protein exprimiert, mit einer Testverbindung; und Bestimmung, ob die Testverbindung die APOBEC4-Transkription und/oder -Expression der Zelle beeinflusst, wobei eine Abnahme der APOBEC4-Transkription und/oder -Expression der Zelle anzeigt, dass die Testverbindung eine antivirale Verbindung ist.

21. Das Verfahren gemäß Anspruch 20, wobei die APOBEC4-Transkription und/oder -Expression einer ersten Zelle in Gegenwart einer Testverbindung verglichen wird mit der APOBEC4-Transkription und/oder -Expression einer zweiten Zelle unter Abwesenheit der Testverbindung, wobei eine Abnahme der APOBEC4-Transkription und/oder -Expression der ersten Zelle verglichen mit der zweiten Zelle anzeigt, dass die Testverbindung eine antivirale Verbindung ist.

22. Das Verfahren aus Anspruch 20 oder 21, wobei die APOBEC4-Transkription und/oder -Expression mittels RT-PCR, Northern-Blotting und/oder Western-Blotting bestimmt wird.

23. Ein Verfahren zur Ausleseprüfung von antiviralen Verbindungen, das Verfahren umfassend die Schritte:
Kontaktieren einer Zelle, die einen Retrovirus und ein APOBEC4-Protein exprimiert, mit einer Testverbindung; und
Bestimmung des Titers des Retrovirus in Gegenwart und unter Abwesenheit der Testverbindung,
wobei eine Abnahme des Titers anzeigt, dass die Verbindung eine antivirale Verbindung ist.

24. Das Verfahren gemäß einem der Ansprüche 20-23, wobei die Testverbindung ein gegen APOBEC4-mRNA gerichtetes siRNA- oder shRNA-Molekül ist.

## Revendications

1. Protéine APOBEC4 humaine modifiée **caractérisée en ce que** l'extrémité N-terminale est modifiée de sorte à stabiliser la protéine APOBEC4 ou l'extrémité C-terminale est modifiée, dans laquelle la modification de l'extrémité N-terminale comprend l'addition d'au moins une séquence d'acides aminés choisie dans le groupe comprenant le marqueur V5, le marqueur c-myc et le marqueur HA ou une séquence d'acides aminés homologue à celle-ci, et dans laquelle la modification de l'extrémité C-terminale comprend l'addition d'au moins une séquence d'acides aminés choisie dans le groupe comprenant le marqueur V5, le marqueur c-myc et le marqueur HA ou une séquence d'acides aminés homologue à celle-ci.

2. Protéine APOBEC4 modifiée selon la revendication 1, dans laquelle l'au moins une séquence d'acides aminés ajoutée à l'extrémité N-terminale est un marqueur HA ou une séquence présentant une identité de séquence d'au moins 90 % avec celle-ci.

3. Protéine APOBEC4 modifiée selon la revendication 1, dans laquelle l'au moins une séquence d'acides aminés ajoutée à l'extrémité C-terminale est un marqueur HA ou 3 marqueurs HA ou une séquence présentant une identité de séquence d'au moins 90 % avec celle-ci.

4. Cellule exprimant la protéine APOBEC4 modifiée selon les revendications 1 à 3.

5. Cellule selon la revendication 4, la cellule produisant en outre un rétrovirus ou des particules virales de celui-ci.

6. Cellule selon la revendication 5, dans laquelle le rétrovirus est un lentivirus et/ou un virus à réplication déficiente.

7. Cellule selon la revendication 6, dans laquelle l'extrémité N-terminale de la protéine APOBEC4 est modifiée de sorte à stabiliser la protéine APOBEC4.

8. Cellule selon les revendications 4 à 7, la cellule étant choisie dans le groupe comprenant une cellule 293T, une cellule HeLa, un lymphocyte T auxiliaire, et un macrophage.

9. Acide nucléique codant pour la protéine APOBEC4 modifiée selon l'une quelconque des revendications 1 à 3 ou le complément de celle-ci.

10. Acide nucléique comprenant la séquence d'acides nucléiques selon la revendication 9.

11. Acide nucléique ou vecteur destiné à une thérapie génique somatique comprenant l'acide nucléique selon la revendication 10, l'acide nucléique codant pour la protéine APOBEC4 étant lié de manière fonctionnelle à un promoteur.

12. Vaccin comprenant la cellule selon la revendication 7 et un transporteur pharmaceutique acceptable.

13. Particule rétrovirale à réplication déficiente comprenant l'acide nucléique selon les revendications 10 et 11, dans laquelle l'acide nucléique est un ARN.

14. Particule rétrovirale selon la revendication 13, la particule rétrovirale étant dérivée des virus VIH, VIH-1, VIH-2, VIS, VISagm, VISmac, spumavirus, VIF, VAIE ou VLM.

15. Procédé *in vitro* destiné à réduire la production de rétrovirus ou de lentivirus ou de particules virales provenant de ceux-ci dans une cellule produisant un rétrovirus, un lentivirus ou des particules virales provenant de ceux-ci, le procédé comprenant l'expression d'une protéine APOBEC4 modifiée selon les revendications 1 et 3 dans ladite cellule, dans lequel l'extrémité C-terminale de la protéine APOBEC4 est modifiée.

16. Procédé selon la revendication 15, dans lequel la production des rétrovirus ou des particules virales provenant de ceux-ci est réduite d'au moins 20 %.

17. Procédé de production de particules rétrovirales, le procédé comprenant la culture d'une cellule selon la revendication 7.

18. Utilisation d'une cellule selon la revendication 7, de l'acide nucléique selon la revendication 11 ou de la particule rétrovirale selon les revendications 13 à 14 pour la fabrication d'un médicament.

19. Utilisation d'une cellule selon la revendication 7, de l'acide nucléique selon la revendication 11 ou de la particule rétrovirale selon les revendications 13 à 14 pour la fabrication d'un médicament destiné à traiter ou à vacciner un patient contre une infection par un rétrovirus.

20. Procédé destiné à cribler à la recherche de composés antiviraux, le procédé comprenant les étapes consistant à :
mettre en contact une cellule exprimant une protéine APOBEC4 humaine avec un composé test ; et
déterminer si le composé test affecte la transcription et/ou l'expression de la protéine APOBEC4 dans ladite cellule,
dans lequel une diminution de la transcription et/ou de l'expression de la protéine APOBEC4 dans ladite cellule indique que le composé test est un composé antiviral.

21. Procédé selon la revendication 20, dans lequel la transcription et/ou l'expression de la protéine APOBEC4 dans une première cellule en présence d'un composé test est(sont) comparée(s) à la transcription et/ou l'expression de la protéine APOBEC4 dans une seconde cellule en l'absence dudit composé test,
dans lequel une diminution de la transcription et/ou de l'expression de la protéine APOBEC4 dans la première cellule par rapport à la seconde cellule indique que le composé test est un composé antiviral.

22. Procédé selon les revendications 20 ou 21, dans lequel la transcription et/ou l'expression de la protéine APOBEC4 est(sont) déterminée(s) par RT-PCR, transfert d'ARN et/ou transfert de Western.

23. Procédé destiné à cribler à la recherche de composés antiviraux, le procédé comprenant les étapes consistant à :
mettre en contact une cellule exprimant un rétrovirus et une protéine APOBEC4 avec un composé test ; et
déterminer le titre du rétrovirus en présence et en l'absence du composé test,
dans lequel une diminution du titre indique que le composé test est un composé antiviral.

24. Procédé selon l'une quelconque des revendications 20 à 23, dans lequel le composé test est une molécule d'ARNsi ou d'ARNsh dirigée contre l'ARNm de la protéine APOBEC4.
